(19) Europäisches Patentamt
European Patent Office
Office européen des brevets

(11) EP 1 185 527 B1

(12) EUROPÄISCHE PATENTSCHRIFT

(45) Veröffentlichungstag und Bekanntmachung des Hinweises auf die Patenterteilung:
**27.07.2005 Patentblatt 2005/30**

(21) Anmeldenummer: **00929466.1**

(22) Anmeldetag: **29.04.2000**

(51) Int Cl.[7]: **C07D 409/10**, A61K 31/415

(86) Internationale Anmeldenummer:
**PCT/EP2000/003891**

(87) Internationale Veröffentlichungsnummer:
**WO 2000/068226 (16.11.2000 Gazette 2000/46)**

(54) **1-(P-THIENYLBENZYL)-IMIDAZOLE ALS AGONISTEN VON ANGIOTENSIN-(1-7)-REZEPTOREN, VERFAHREN ZU IHRER HERSTELLUNG, IHRE VERWENDUNG UND SIE ENTHALTENDE PHARMAZEUTISCHE PRÄPARATE**

1-(P-THIENYLBENZYL)-IMIDAZOLES AS ANGIOTENSIN-(1-7) RECEPTOR AGONISTS, METHOD FOR THE PRODUCTION AND THE UTILIZATION THEREOF AND PHARMACEUTICAL PREPARATIONS CONTAINING SAID COMPOUNDS

1(P-THIENYLBENZYL)-IMIDAZOLES COMME AGONISTES DES RECEPTEURS DE L'ANGIOTENSINE (1-7), LEUR PROCEDE DE PRODUCTION, LEUR UTILISATION, ET PREPARATIONS PHARMACEUTIQUES LES CONTENANT

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **05.05.1999 DE 19920815**
**21.12.1999 DE 19961686**

(43) Veröffentlichungstag der Anmeldung:
**13.03.2002 Patentblatt 2002/11**

(73) Patentinhaber: **Aventis Pharma Deutschland GmbH**
**65929 Frankfurt am Main (DE)**

(72) Erfinder:
- **HEITSCH, Holger**
**D-55252 Mainz-Kastel (DE)**
- **WIEMER, Gabriele**
**D-61476 Kronberg (DE)**

(56) Entgegenhaltungen:
**EP-A- 0 512 675** **WO-A-94/27597**

- **H. YANAGISAWA ET. AL.: "Nonpeptide Angiotensin II Antagonists. Synthesis, Biological Activities, and Structure-Activity Relationships of Imidazole-5-Carboxylic Acids Bearing Alkyl, Alkenyl and Hydroxyalkyl Substitutents at the 4-Position and Their Related Compounds. " JOURNAL OF MEDICINAL CHEMISTRY, Bd. 39, Nr. 1, 5. Januar 1996 (1996-01-05), Seiten 323-38, XP002144317**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 185 527 B1

Printed by Jouve, 75001 PARIS (FR)

## Beschreibung

Die Erfindung betrifft neue 1-(p-Thienylbenzyl)-Imidazole der Formel (I),

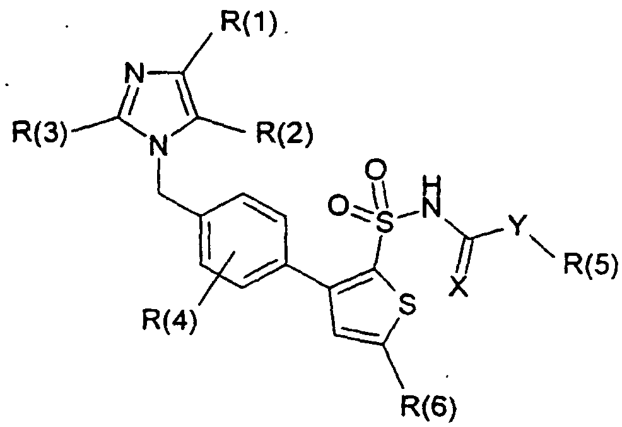

(I)

die potente Agonisten von Angiotensin-(1-7)-Rezeptoren sind und durch die mit der Stimulation dieser Rezeptoren an Endothelzellen verbundene Produktion und Freisetzung der vasorelaxierenden, antithrombotischen und kardioprotektiven Botenstoffe cyclisches 3',5'-Guanosinmonophoshat (cGMP) und Stickstoffmonoxid (NO) wertvolle Arzneimittel zur Behandlung und Prophylaxe von Bluthochdruck, Herzhypertrophie, Herzinsuffizienz, koronaren Herzerkrankungen wie der Angina Pectoris, Herzinfarkt, vaskulärer Restenose nach Angioplastie, Kardiomyopathien, einer endothelialen Dysfunktion bzw. endothelialer Schädigungen, z.B. als Folge atheriosklerotischer Prozesse oder beim Diabetis mellitus, sowie von arterieller und venöser Thrombose darstellen.

In den Anmeldungen EP-A 512675 und WO 94/27597 sind Thienyl-benzylsubstituierte imidazole als Angiotensin II-Rezeptor-Antagonisten und deren Verwendung zur Behandlung der Hypertension, Herzinsuffizienz, Migräne, Alzheimerschen Krankheit und als Antidepressiva beschrieben. Darüber hinaus sind Thienylbenzyl-substituierte Imidazo-pyridine aus EP-A 513979 als Antagonisten von Angiotensin II-Rezeptoren und deren Verwendung zur Behandlung der Hypertension, Herzinsuffizienz, Migräne und Alzheimerscher Krankheit sowie aus US-5444067 als Angiotensin II-Agonisten und deren Verwendung zur Behandlung der Hypotension und des Hypoaldosteronismus bekannt. Des weiteren sind aus EP-A 534706 Thienylbenzyl-substituierte Chinazolinone und Pyridopyrimidone und aus EP-A 510812 Thienylbenzyl-substituierte Triazole als Antagonisten von Angiotensin II-Rezeptoren bekannt.

Die hier beschriebenen 1-(p-Thienylbenzyl)-Imidazole der Formel (I) und deren Verwendung als Agonisten von Angiotensin-(1-7)-Rezeptoren sind dabei in den angeführten Anmeldungen weder beschrieben, vorweggenommen noch nahegelegt.

Überraschend wurde gefunden, daß 1-(p-Thienylbenzyl)-Imidazole der Formel (I) eine ausgeprägte Wirkung auf Angiotensin-(1-7)-Rezeptoren haben und die biologische Wirkung des Effektorhormons Angiotensin-(1-7) mimikrieren.

Gegenstand der Erfindung sind somit Verbindungen der Formel (I),

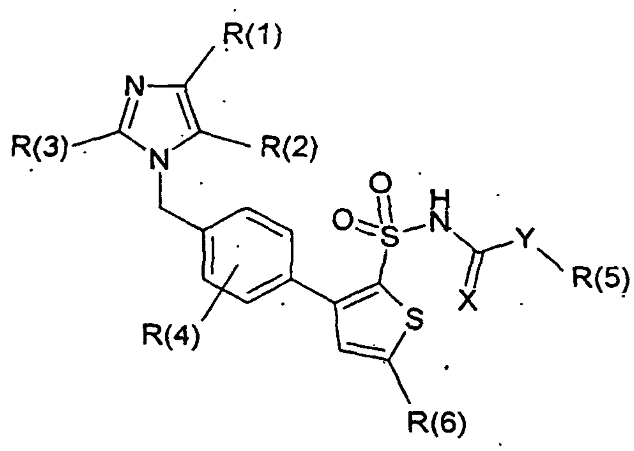

(I)

in denen die angeführten Reste die folgende Bedeutung haben:

R(1)
1. Halogen;
2. Hydroxy;
3. ($C_1$-$C_4$)-Alkoxy;
4. ($C_1$-$C_8$)-Alkoxy, wobei 1 bis 6 Kohlenstoffatome gegen die Heteroatome O, S oder NH, vorzugsweise gegen O ausgetauscht sind;
5. ($C_1$-$C_4$)-Alkoxy, substituiert mit einem gesättigtem cyclischen Ether wie Tetrahydropyran oder Tetrahydrofuran;
6. O-($C_1$-$C_4$)-Alkenyl;
7. O-($C_1$-$C_4$)-Alkyl-Aryl; und
8. Aryloxy, unsubstituiert oder substituiert mit einem Substituenten aus der Reihe Halogen, ($C_1$-$C_3$)-Alkyl, ($C_1$-$C_3$)-Alkoxy oder Trifluormethyl;

R(2)
1. CHO;
2. COOH; und
3. CO-O-($C_1$-$C_4$)-Alkyl;

R(3)
1. ($C_1$-$C_4$)-Alkyl; und
2. Aryl;

R(4)
1. Wasserstoff;
2. Halogen, und
3. ($C_1$-$C_4$)-Alkyl;

X
1. Sauerstoff;
2. Schwefel;

Y
1. Sauerstoff; und
2. -NH-;

R(5)
1. Wasserstoff;
2. ($C_1$-$C_6$)-Alkyl; und
3. ($C_1$-$C_4$)-Alkyl-Aryl;

wobei R(5) nur dann auch Wasserstoff sein kann, wenn Y die unter 2. angeführte Bedeutung besitzt;

R(6)
1. ($C_1$-$C_5$)-Alkyl;

in allen ihren stereoisomeren Formen und Mischungen davon in allen Verhältnissen, und ihre physiologisch verträglichen Salze;

ausgenommen Verbindungen der Formel (1), in denen gleichzeitig R(1) für Halogen steht und R(2) die unter 2. und 3. angeführte Bedeutung hat.

Der Begriff Alkyl bedeutet, sofern nicht anders angegeben, geradkettige oder verzweigte gesättigte Kohlenwasserstoffreste. Dies gilt auch für davon abgeleitete Substituenten wie Alkoxy oder den Rest $S(O)_m$-Alkyl. Beispiele für Alkylreste sind Methyl, Ethyl, n-Propyl, Isopropyl, Isobutyl, sec-Butyl, tert-Butyl, n-Pentyl, n-Hexyl. Beispiele für Alkoxy sind Methoxy, Ethoxy, n-Propoxy, Isopropoxy. Beispiele für Aryloxy sind Phenoxy oder Naphthoxy. Bevorzugt ist Phenoxy.

Alkenyl steht für einfach oder mehrfach ungesättigte Kohlenwasserstoffreste, in denen sich die Doppelbindungen in beliebigen Positionen befinden können. Beispiele für Alkenyl sind Vinyl, Propenyl und Butenyl.

Halogen steht für Fluor, Chlor, Brom oder Jod, vorzugsweise für Chlor oder Fluor.

Aryl steht für Phenyl oder Naphthyl, bevorzugt Phenyl.

In substituierten Arylresten können sich die Substituenten in beliebigen Positionen zueinander befinden.

Beispiele für Arylalkylreste sind Phenylmethyl (Benzyl), Phenylethyl, Phenylpropyl, Phenylbutyl, Naphthylmethyl, Naphthylethyl, Naphthylpropyl, Naphthylbutyl.

Enthalten die Verbindungen der Formel (I) eine oder mehrere saure oder basische Gruppen so sind auch die entsprechenden physiologisch verträglichen Salze Gegenstand der Erfindung, insbesondere die pharmazeutisch verwendbaren Salze. So können die Verbindungen der Formel (I), die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel (I), die eine oder mehrere basische, d. h. protonierbare, Gruppen tragen, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel (I) gleichzeitig saure und basische Gruppen im Molekül, so gehören neben den geschilderten Salzformen auch innere Salze, sogenannte Betaine, zu der Erfindung. Salze können aus den Verbindungen der Formel (I) nach üblichen Verfahren erhalten werden, beispielsweise durch Vereinigung mit einer Säure bzw. Base in einem Lösungs- oder Dispergiermittel oder auch durch Anionenaustausch aus anderen Sätzen.

Unter physiologisch verträglichen Salzen von Verbindungen der Formel (I) sind beispielsweise auch organische und anorganische Salze zu verstehen , wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B: Essigsäure, Zitronensäure, Benzoesäure, Maleinsäure, Fumarsäure, Weinsäure und p-Toluolsulfonsäure bevorzugt.

Die vorliegende Erfindung umfaßt weiterhin Solvate von Verbindungen der Formel (I), zum Beispiel Hydrate oder Addukte mit Alkoholen, sowie Derivate der Verbindungen der Formel (I) wie zum Beispiel Ester, und Pro-Drugs und aktive Metabolite.

Bevorzugt sind Verbindungen der Formel (I), in denen

R(1)
1. Chlor;
2. Hydroxy;
3. Methoxy, Ethoxy, Propyloxy;
4. Methoxyethoxy, Methoxypropoxy;
5. Allyloxy; und
6. Phenoxy;

R(4)
1. Wasserstoff; und
2. Chlor;

R(5)
1. Wasserstoff; und
2. $(C_1-C_4)$-Alkyl;

R(6) n-Propyl und 2-Isobutyl;

bedeuten und die übrigen Reste wie oben definiert sind, in allen ihren stereoisomeren Formen und Mischungen davon, und ihre physiologisch verträglichen Salze.

Weiterhin sind Verbindungen der Formel (I) bevorzugt, in denen

R(1) Halogen, bevorzugt Chlor; $(C_1-C_4)$-Alkoxy, bevorzugt Methoxy, Ethoxy, Propyloxy, besonders bevorzugt Me-

thoxy; oder ($C_1$-$C_8$)-Alkoxy, wobei 1 bis 6 Kohlenstoffatome gegen die Heteroatome O, S oder NH vorzugsweise O ausgetauscht sind, bevorzugt Methoxyethoxy oder Methoxypropoxy;

R(2) CHO;

R(3) Aryl, bevorzugt Phenyl;

R(4) Halogen, bevorzugt Chlor, oder Wasserstoff;

R(5) ($C_1$-$C_6$)-Alkyl, bevorzugt Methyl, Ethyl, Propyl, Butyl;

R(6) ($C_1$-$C_5$)-Alkyl, bevorzugt Ethyl, Propyl oder Butyl;

X Sauerstoff;

Y Sauerstoff oder -NH-; bedeutet,

in allen ihren stereoisomeren Formen und Mischungen davon, und ihre physiologisch verträglichen Salze.

Ganz besonders bevorzugt sind Verbindungen der Formel (I), wenn diese Verbindungen der Formel (II) darstellen,

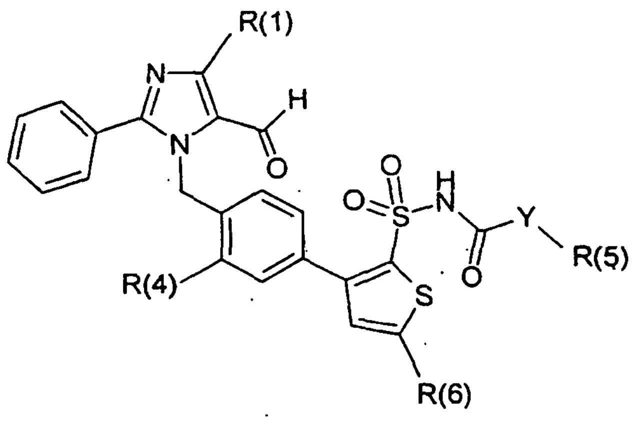

(II)

in der die Reste R(1), R(4), R(5), R(6) und Y die oben angeführte Bedeutung haben, in allen ihren stereoisomeren Formen und Mischungen davon, und ihre physiologisch verträglichen Salze.

Bevorzugt sind auch Verbindungen der Formel (I), in denen R(1 ) ($C_1$-$C_4$)-Alkoxy oder ($C_1$-$C_8$)-Alkoxy, wobei 1 bis 6 Kohlenstoffatome gegen die Heteroatome O, S oder NH, vorzugsweise O ausgetauscht sind bedeutet und die übrigen Reste wie oben definiert sind, in allen ihren stereoisomeren Formen und Mischungen davon, und ihre physiologisch verträglichen Salze.

Besonders bevorzugt sind auch Verbindungen der Formel (I), in denen R(2) CHO bedeutet und die übrigen Reste wie oben definiert sind, in allen ihren stereoisomeren Formen und Mischungen davon, und ihre physiologisch verträglichen Salze.

Bevorzugt sind weiterhin Verbindungen der Formel (1), in denen X O bedeutet und die übrigen Reste wie oben definiert sind, in allen ihren stereoisomeren Formen und Mischungen davon, und ihre physiologisch verträglichen Salze.

Als besonders bevorzugte Verbindungen der Formel (I) seien genannt:

4-Chloro-5-formyl-2-phenyl-1-[[4-[2-(n-butyloxycarbonylsulfonamido)-5-isobutyl-3-thienyl]-phenyl]-methyl]-imidazol;

5-Formyl-4-methoxy-2-phenyl-1-[[4-[2-(n-butyioxycarbonylsulfonamido)-5-isobutyi-3-thienyl]-phenyl]-methyl]-imidazol;

5-Formyl-4-methoxy-2-phenyl-1-[[4-[2-(n-propyloxycarbonylsulfonamido)-5-isobutyl-3-thienyl]-phenyl]-methyl]-imidazol

5-Formyl-4-methoxy-2-phenyl-1-[[4-[2-(ethoxycarbonylsulfonamido)-5-isobutyl-3-thienyl]-phenyl]-methyl]-imidazol;

5-Formyl-4-methoxy-2-phenyl-1-[[4-[2-(methoxycarbonyfsulfonamido)-5-isobutyl-3-thienyl]-phenyl]-methyl]-imidazol;

5-Formyl-4-methoxy-2-phenyl-1-[[4-[2-(n-butylaminocarbonylsulfonamido)-5-isobutyl-3-thienyl]-phenyl]-methyl]-imidazol;

5-Formyl-4-methoxy-2-phenyl-1-[[4-[2-(ethylaminocarbonylsulfonamido)-5-isobutyl-3-thienyl]-phenyl]-methyl]-imidazol;

5-Formyl-4-methoxy-2-phenyl-1-[[4-[2-(ethylaminocarbonylsulfonamido)-5-isobutyl-3-thienyl]-phenyl]-methyl]-imidazol Natriumsalz;

5-Formyl-4-methoxy-2-phenyl-1-[[4-[2-(ethylaminocarbonylsulfonamido)-5-isobutyl-3-thienyl]-phenyl]-methyl]-imidazol L-Lysin-Salz;

5-Formyl-4-methoxy-2-phenyl-1-[[4-[2-(ethylaminocarbonylsulfonamido)-5-isobutyl-3-thienyl]-phenyl]-methyl]-imidazol Tris(hydroxymethyl)aminomethan-Salz;

5-Formyl-4-methoxy-2-phenyl-1-[[4-[2-(methylaminocarbonylsulfonamido)-5-isobutyl-3-thienyl]-phenyl]-methyl]-imidazol;

5-Formyl-4-methoxyethoxy-2-phenyl-1-[[4-[2-(n-butyloxycarbonylsulfonamido)-5-isobutyl-3-thienyl]-phenyl]-methyl]-imidazol;

5-Formyl-4-methoxy-2-phenyl-1-[[4-[2-(n-butyloxycarbonylsulfonamido)-5-isobutyl-3-thienyl]-2-chlorphenyl]-methyl]-imidazol;

5-Formyl-4-methoxy-2-phenyl-1-[[4-[2-(n-butyloxycarbonylsulfonamido)-5-isobutyl-3-thienyl]-2-chlorphenyl]-methyl]-imidazol;

4-Chloro-5-formyl-2-phenyl-1-[[4-[2-(n-butyloxycarbonylsulfonamido)-5-n-propyl-3-thienyl]-phenyl]-methyl]-imidazol;

5-Formyl-4-methoxy-2-phenyl-1-[[4-[2-(n-butyloxycarbonylsulfonamido)-5-n-propyl-3-thienyl]-phenyl]-methyl]-imidazol;

5-Formyl-4-methoxy-2-phenyl-1-[[4-[2-(methoxycarbonylsulfonamido)-5-n-propyl-3-thienyl]-pheriyl]-methyl]-imidazol;

5-Formyl-4-methoxy-2-phenyl-1-[[4-[2-(n-butylaminocarbonylsulfonamido)-5-n-propyl-3-thienyl]-phenyl]-methyl]-imidazol; oder

5-Formyl-4-methoxy-2-phenyl-1-[[4-[2-(methylaminocarbonylsulfonamido)-5-n-propyl-3-thienyl]-phenyl]-methyl]-imidazol; sowie deren physiologisch verträglichen Salze.

Die Erfindung betrifft weiterhin Verfahren zur Herstellung der Verbindungen der Formel (I), die durch die im folgenden wiedergegebenen Reaktionsschritte gekennzeichnet sind:

a) 4-Chlor-5-formyl-imidazol-Derivate der Formel (III),

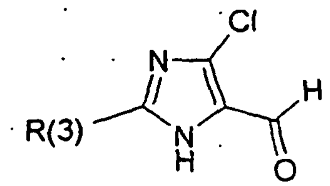

(III)

in der R(3) die oben angeführte Bedeutung besitzt und deren Herstellung beispielsweise in Chem. Pharm. Bull. 24, 1976, 960-969 beschrieben ist, werden mit p-Brom-benzylbromiden der Formel (IV),

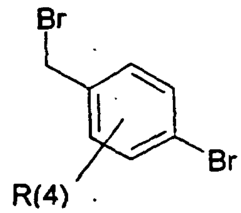

(IV)

in denen R(4) wie oben definiert ist, zu Verbindungen der Formel (V),

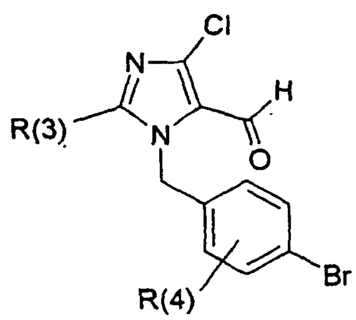

(V)

in denen R(3) und (R4) die oben angeführte Bedeutung besitzen, umgesetzt wobei die Alkylierung in Gegenwart einer organischen oder anorganischen Base wie beispielsweise Triethylamin, $K_2CO_3$ oder $Cs_2CO_3$ in einem inerten Lösungsmittel wie beispielsweise DMF durchgeführt werden kann. Verbindungen der Formel (IV) sind käuflich erhältlich oder können nach an sich bekannten Methoden hergestellt werden.

b) Die Verbindungen der Formel (V) können mit Thiophen-3-boronsäuren der Formel (VI).

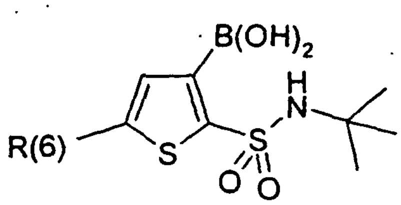

(VI)

in denen R(6) wie oben definiert ist und deren Herstellung aus EP-A 512 675 bekannt ist, zu den 1-(p-Thienyl)-Imidazolen der Formel (VII),

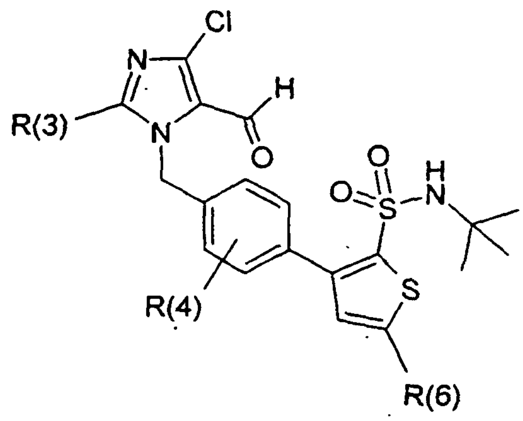

(VII)

in denen R(3), R(4) und R(6) wie oben definiert sind, umgesetzt werden. Diese Suzuki-Typ Cross-coupling-Reaktion erfolgt vorzugsweise unter Verwendung von Palladium(II)acetat und. Triphenylphosphin oder Tetrakistriphenylphosphinpalladium als Katalysatoren in Gegenwart einer Base wie z.B. Cäsium- oder Kaliumcarbonat beispielsweise in Lösungsmittelgemischen aus Ethanol und Toluol bei Temperaturen bis zum Siedepunkt der Lösungsmittel; entsprechende Reaktionen werden zum Beispiel in Synthetic Commun. 11 (1981) 513, J. Med. Chem. 38 (1995) 2357-2377 und Liebigs Ann. 1995, 1253-1257 beschrieben.

c) Die Verbindungen der Formel (VII) können durch Abspaltung der tert.-Butyl-Schutzgruppe in die Sulfonamide der Formel (VIII)

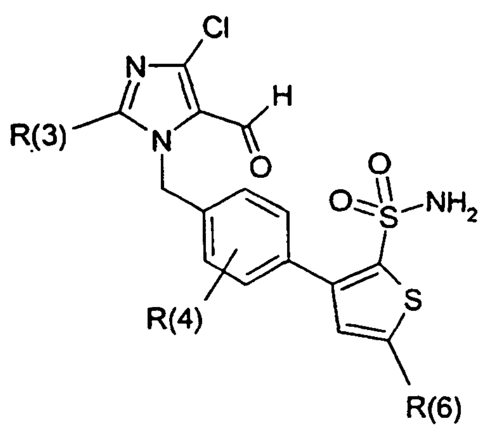

(VIII)

in denen R(3), R(4) und R(6) wie oben definiert sind, überführt werden. Diese Abspaltung erfolgt bevorzugt durch Behandlung der Verbindungen der Formel (VII) mit organischen Säuren wie beispielsweise konzentrierter Trifluoressigsäure in Gegenwart von Anisol.

d) Die Verbindungen der Formel (VIII) können durch Substitution des Chloratoms in Position 4 des Imidazol-Ringes in die Verbindungen der Formel (IX),

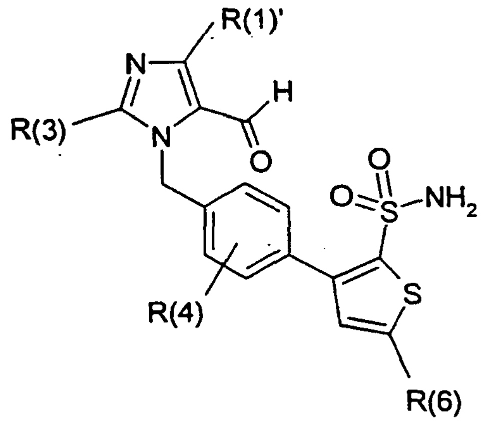

(IX)

in der R(3), R(4) und R(6) wie oben definiert sind und R(1)' für die unter 2. bis 8. angeführten Reste steht, überführt werden. Diese Substitution des Chloratoms kann dabei beispielsweise durch Behandlung der Verbindungen der Formel (VIII) mit Alkoholaten erfolgen, die in situ durch Einwirkung von Basen wie NaOH oder NaH auf die im allgemeinen auch als Lösungsmittel verwendeten Alkohole wie beispielsweise Methanol, Ethanol oder Ethylenglycolmonomethylether bei Temperaturen von 50 °C bis zum Siedepunkt der Alkohole gebildet werden. Alternativ können die Verbindungen der Formel (IX), in denen R(1)' für ($C_1$-$C_4$)-Alkoxy steht, über eine Etherspaltung durch Behandlung vorzugsweise der Methoxyether der Formel (IX) mit konzentrierten Säuren wie HI und HBr oder mit Lewissäuren wie $BF_3$, $BCl_3$, $BBr_3$, $AlCl_3$ oder deren Etherate, vorzugsweise mit $BBr_3$, in einem inerten Lösungsmittel wie beispielsweise $CH_2Cl_2$ in die korrespondierenden Phenole überführt werden, die dann anschließend mit den geeignet substituierten Halogeniden wie zum Beispiel (2-Bromethyl)-methylether oder Benzylbromid in Gegenwart einer Base in einem inerten Lösungsmittel bei Temperaturen bis zum Siedepunkt des Lösungsmittels nach an sich bekannten Verfahren umgesetzt werden können.
Die entsprechenden Diphenylether-Verbindungen können aus dem Umsatz der Phenole der Formel (IX) mit Boronsäuren wie beispielsweise Phenylboronsäure oder 4-Methoxyphenylboronsäure in Gegenwart von Kupferkatalysatoren wie beispielsweise $Cu(OAc)_2$ erhalten werden; entsprechende Reaktionen sind beispielsweise in Tetrahedron Lett. 39 (1998), 2937-2940 beschrieben.

e) Aus den Sulfonamiden der Formel (IX) können durch Umsatz mit R(5)-substituierten Chlorameisensäureestern die Sulfonylurethane der Formel (Ia),

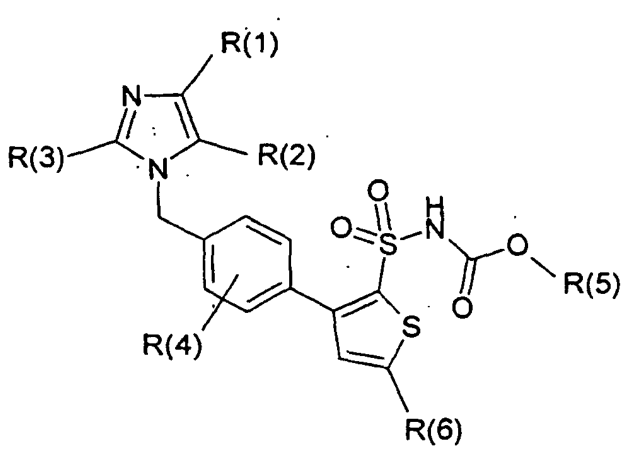

(Ia)

in der (R1), R(2), R(3), R(4), R(6) wie oben definiert sind und R(5) nur die unter 2. und 3. angeführte Bedeutung besitzt, hergestellt werden. Diese Umsetzung kann in Gegenwart einer Base wie beispielsweise Pyridin und eines Acylierungsbeschleunigers wie 4-Pyrrolidino-pyridine bei Temperaturen von RT bis 150°C, vorzugsweise jedoch bei RT durchgeführt werden.

f) Aus den Sulfonamiden der Formel (IX) können durch Behandlung mit R(5)-substituierten Isocyanaten bzw. Isothiocyanaten die Sulfonylharnstoffe der Formel (Ib),

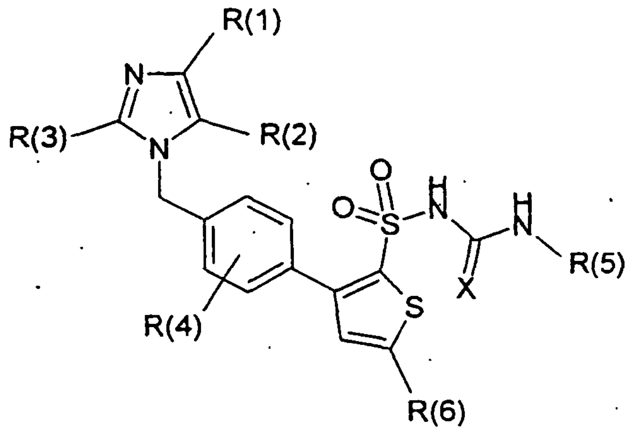

(Ib)

in der (R1), R(2), R(3), R(4), R(6) und X wie oben definiert sind und R(5) nur die unter 2. und 3. angeführte Bedeutung besitzt, gewonnen werden. Die Umsetzung mit den R(5)-substituierten Isocyanaten und -Isothiocyanaten kann in Gegenwart einer Base in einem inerten Lösungsmittel bei Temperaturen von RT bis 150°C durchgeführt werden.

Als Basen eignen sich z.B. Alkalimetall- oder Erdalkalimetallhydroxide, -hydride, - amide oder -alkoholate, wie Natriumhydroxid, Kaliumhydroxid, Calciumhydroxid, Natriumhydrid, Kaliumhydrid, Calciumhydrid, Natriumamid, Kaliumamid, Natriummethylat, Natriumethylat oder Kalium-*tert*.-butylat. Als inerte Lösungsmittel eignen sich Ether wie THF, Dioxan, Ethylenglykoldimethylether oder Diglyme, Ketone wie Aceton oder Butanon, Nitrile wie Acetonitril, Nitroverbindungen wie Nitromethan, Ester wie Ethylacetat, Amide wie DMF oder N-Methylpyrrolidon, Hexamethylphosphorsäuretriamid, Sulfoxide wie DMSO und Kohlenwasserstoffe wie Benzol, Toluol oder Xylole. Weiterhin eignen sich auch Gemische dieser Lösungsmittel untereinander.

Die Sulfonylharnstoffe der Formel (Ib) sind auch durch Umsetzung von Aminen R(5)-$NH_2$ mit Sulfonylisocyanat-Derivaten, die aus den Sulfonamiden der Formel (IX) beispielsweise durch Behandlung mit Phosgen oder einem Phosgenersatzstoff wie Triphosgen resultieren, herstellbar. Die Sulfonylharnstoffe der Formel (Ib) lassen sich alternativ auch durch Umsetzung der Sulfonamide der Formel (IX) mit 2,2,2-Trichloracetamid-Derivaten eines geeigneten Amins R(5)-$NH_2$ in Gegenwart einer Base in einem inerten, hochsiedenden Lösungsmittel wie z.B. DMSO oder aus den durch Umsatz mit Chlorameisäureethylester zugänglichem entsprechenden Sulfonylurethan der Formel (la) durch Einwirkung des entsprechenden Amins R(5)-$NH_2$ in einem inerten, hochsiedenden Lösungsmittel wie z.B. Toluol bei Temperaturen bis zum Siedepunkt des jeweiligen Lösungsmittels darstellen, welches beispielsweise in J. Med. Chem. 38 (1995) 2357-2377 und in Bioorg. Med. Chem. 5 (1997) 673-678 beschrieben ist.

Die Herstellung der N-unsubstituierten Sulfonylharnstoffe der Formel (Ib), in denen R(5) für Wasserstoff steht, kann durch Verseifung der nach Umsatz der Sulfonamide der Formel (IX) mit Bromcyan in der Gegenwart von $K_2CO_3$ in Acetontril resultierenden Sulfonamidonitrile mit Schwefelsäure bei Temperaturen -10 - 0 °C erfolgen.

Nach an sich bekannten Methoden, wie sie in der Literatur (z.B. in den Standardwerken wie Houben-Weyl, Methoden der Organischen Chemie, Georg Thieme Verlag, Stuttgart, Organic Reactions, John Wiley & Sons, Inc., New York oder Larock, Comprehensive Organic Transformations, VCH, Weinheim) können durch Oxidation der Aldehyd-Gruppe in den Verbindungen der Formel (I) dann die entsprechenden Carbonsäuren bzw. Carbonsäureestern der Formel (I) hergestelltwerden.

Die Erfindung betrifft auch Verbindungen der Formel (X)

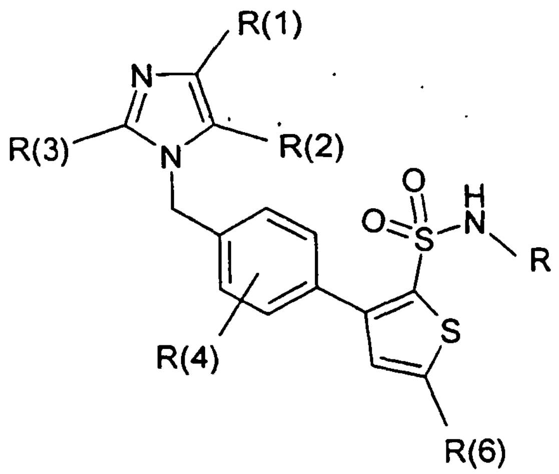

(X)

in denen R Wasserstoff oder eine geeignete Schutzgruppe wie beispielsweise ($C_1$-$C_6$)-Alkyl, bevorzugt tert.-Butyl bedeutet und die Reste R(1), R(2), R(3), R(4), R(6) wie oben definiert sind, in allen ihren stereoisomeren Formen und Mischungen davon, und ihre physiologisch verträglichen Salze.

Die Verbindungen der Formel (X) stellen wertvolle Zwischenprodukte zur Herstellung der erfindungsgemäßen Verbindungen der Formel (I) dar. Desweiteren weisen die Verbindungen der Formel (X) eine hohe Affinität zum Angiotensin(1-7)-Rezeptor auf und können als Angiotensin(1-7)-Rezeptoragonisten und somit als Arzneimittel zur Behandlung und/oder Prophylaxe von Krankheiten, die primär oder sekundär durch eine reduzierte Produktion und/oder Freisetzung der vasorelaxierenden, antithrombotischen und kardioprotektiven Botenstoffe cyclisches 3',5'-Guanosinmonophoshat (cGMP) und Stickstoffmonoxid (NO) verursacht oder zumindest mitverursacht werden, zum Beispiel zur Behandlung und/oder Prophylaxe von Bluthochdruck, Herzhypertrophie, Herzinsuffizienz, koronaren Herzerkrankungen wie der Angina Pectoris, Herzinfarkt, vaskulärer Restenose nach Angioplastie, Kardiomyopathien, einer endothelialen Dysfunktion bzw. endothelialer Schädigungen, z.B. als Folge atheriosklerotischer Prozesse oder beim Diabetis mellitus, sowie von arterieller und venöser Thrombose verwendet werden.

Das vaskuläre Endothelium ist ein metabolisch aktives Organ mit einer Vielzahl regulatorischer Funktionen, daß zur Synthese und Freisetzung vasoaktiver Substanzen befähigt ist. Eine Dysfunktion der gefäßauskleidenden Endothelschicht wird mit der Pathogenese verschiedener kardiovaskulärer Erkrankung wie Artheriosklerose und Hypertension korreliert (Eur. J. Clin. Invest. 1993, 23, 670-685). Eine endotheliale Dysfunktion ist durch eine reduzierte Synthese und/oder Freisetzung der vasorelaxierenden, vasoprotektiven, antithrombotisch und antiproliferative wirksamen Botenstoffe NO und cGMP charakterisiert, die eine wesentliche Rolle in der Prevention und Regression des vaskulären Remodelling und der arteriellen Hypertension spielen. Substanzen, die in der Lage sind, die Synthese und Freisetzung dieser Botenstoffe zu stimulieren, sind daher wertvolle Arzneimittel zur Behandlung aller Krankheiten, die durch eine endotheliale Dysfunktion gekennzeichnet sind.

Durch eine Vielzahl von publizierten Experimenten wurde belegt, daß ein Abbauprodukt des Renin-Angiotensin-Systems, das Heptapeptid Angiotensin-(1-7), ein potentes, endogenes Effektorhormon des Renin-Angiotensin-Systems ist (Hypertension 1991, 18 [Suppl III]: III-126-III133), dessen biologische Wirkung durch die Stimulation spezifischer Rezeptoren, die bevorzugt Angiotensin-(1-7) binden, hervorgerufen wird (Peptides 1993, 14,679-684, Hypertension 1997, 29 [part 2]: 388-393)). Diese Wirkung ist in vielen Fällen der des vasokonstriktorischen Hormons Angiotensin II entgegengerichtet bzw. steht dieser gegenregulatorisch gegenüber (Hypertension 1997, 30 [part 2]: 535-541, Regulatory Peptides 1998, 78, 13-18). In Hypertension 1992, 19 [Suppl. II]: II-49-II-55 und in Am. J. Cardiol. 1998, 82, 17S-19S wurde aufgezeigt, daß Angiotensin-(1-7) die Produktion und/oder die Freisetzung von NO/cGMP und der Prostaglandine $E_2$ und $I_2$ stimuliert, welches durch Vorbehandlung mit $AT_1$- und $AT_2$-Rezeptor-Antagonisten nicht blokkiert wird. In Hypertension 1996, 27 [part 2]: 523-528 wurde eine endothelabhängige Relaxation an intakten Koronararterien von Hunden und Schweinen sowie in J. Cardiovasc. Pharmacol. 1997, 30, 676-682 eine endothelabhängige Relaxation intakter, durch KCl vorkontrahierter Rattenaorten durch Angiotensin-(1-7) beschrieben, die durch $AT_1$-Rezeptor-Antagonisten nicht beeinflußt wird. In Peptides 1993, 14, 679-684 und in Am. J. Physiol. 1995, 269: H313-H319 wurde die blutdrucksenkende Wirkung von Angiotensin-(1-7) bei Dauerinfusion über eine osmotische Minipumpe in spontan hypertensiven Ratten aufgezeigt, wobei Angiotensin-(1-7) in normotensiven Ratten in gleicher Dosis keine Wirkung auf den Blutdruck hatte. Komplementär zu diesen Untersuchungen wurde in Hypertension 1998, 31: 699-705 demonstriert, daß die Infusion eines Angiotensin-(1-7)-Antikörpers dem mittleren arteriellen Blutdruck in wachen, spontan hypertensiven Ratten, die mit Lisinopril und Losartan vorbehandelt waren, erhöht. In Am. J. Hypertension 1998,

11: 137-146 wurde demonstriert, daß in Menschen mit essentieller Hypertonie deutlich niedrigere Plasmaspiegel von Angiötensin-(1-7) nachweisbar sind als in normotensiven Menschen.
In Hypertension 1996, 28, 104-108 wurde die anti-proliferative Wirkung von Angiotensin-(1-7) auf vaskuläre Glattmuskelzetlen und in Hypertension 1999, 33 [part II]: 207-211 die Hemmung der Proliferation von Glattmuskelzellen nach vaskulärer Gewebsschädigung belegt.
Darüberhinaus zeigte Angiotensin-(1-7) in Kochsalz-beladenen, anästhesierten normotensiven Wistar-Ratten auch renale Effekte wie eine erhöhte Natriurese und Diurese (Am. J. Physiol. 1996, 270, F141-F147).

Die hier beschriebenen Verbindungen der Formel (I) sind potente, nicht-peptidische-Agonisten der postulierten Angiotensin-(1-7)-Rezeptoren, die vorzugsweise in den Gefäßen (einschließlich Endothel), in der Niere, im ZNS und im Herz lokalisiert sind: Sie mimikriieren daher die vorstehend beschriebene, dem Angiotensin II entgegengerichtete biologische Wirkung des Peptidhormons Angiotensin-(1-7), die auf die Produktion und/oder Freisetzung von cGMP und NO aus dem Endothel zurückzuführen ist, ohne dabei dem raschen metabolischen Abbau dieses Hormons zu unterliegen. Durch die Stimulation der Produktion und/oder Freisetzung dieser vasorelaxierenden, antithrombotischen und kardioprotektiven Botenstoffe sind die beschriebenen Angiotensin-(1-7)-Rezeptor-Agonisten der Formel (I) daher Wertvolle Arzneimittel zur Behandlung und/oder Prophylaxe von Krankheiten, die primär oder sekundär durch eine reduzierte Produktion und/oder Freisetzung der vasorelaxierenden, antithrombotischen und kardioprotektiven Botenstoffe cyclisches 3',5'-Guanosinmonophoshat (cGMP) und Stickstoffmonoxid (NO) verursacht oder zumindest mitverursacht werden und somit beispielsweise in der Behandlung und/oder Prophylaxe von Bluthochdruck, Herzhypertrophie, Herzinsuffizienz, koronaren Herzerkrankungen wie der Angina Pectoris, Herzinfarkt, vaskulärer Restenose nach Angioplastie, Kardiomyopathien, einer endothelialen Dysfunktion bzw. endothelialer Schädigungen, z.B. als Folge atheriosklerotischer Prozesse oder beim Diabetis mellitus, sowie von arterieller und venöser Thrombose eingesetzt werden können.

Die Stimulation endothelialer Angiotensin-(1-7)-Rezeptoren durch die Agonisten der Formel (I) verursacht die Freisetzung vasodilatorischer und orginprotektiver Autacoide. Dieser Mechanismus unterscheidet sich dabei vom dem der ACE-Hemmung und $AT_1$-Rezeptor-Blockade durch die Vermeidung entweder von emiedrigtem Gewebs-Angiotensin II (bei ACE-Hemmem) oder von zur Zeit noch nicht abzuschätzender Effekten, die mit erhöhten ANG II-Plasmawerten (bei $AT_1$-Rezeptor-Antagonisten) verbunden sind.

Die Verbindungen der Formel (I) und ihre physiologisch verträglichen Salze können somit am Tier, bevorzugt am Säugetier, und insbesondere am Menschen als Arzneimittel für sich allein, in Mischungen untereinander oder zusammen mit anderen Wirkstoffen verwendet werden, insbesondere in Form von pharmazeutischen Präparaten. Gegenstand der vorliegenden Erfindung ist daher die Verwendung von Verbindungen der Formel (I) und/oder ihren physiologisch verträglichen Salzen zur Herstellung eines Medikaments zur Therapie oder Prophylaxe der vorstehend genannten Krankheitsbilder, sowie pharmazeutische Präparate, die eine wirksame Dosis mindestens einer Verbindung der Formel (I) und/oder eines physiologisch verträglichen Salzes davon als aktiven Bestandteil neben üblichen, pharmazeutisch einwandfreien Trägerstoffen und/oder Hilfsstoffen enthalten. Die pharmazeutische Zubereitungen können für eine enterale oder eine parenterale Anwendung bestimmt sein und enthalten normalerweise 0.5 bis 90 Gewichtsprozent der Verbindung der Formel (I) und/oder ihrer physiologisch verträglichen Salze. Die Menge an Wirkstoff der Formel (I) und/oder dessen physiologisch verträglichen Salzen in den pharmazeutischen Präparaten beträgt im allgemeinen 0.2 bis 500 mg, vorzugsweise 1 bis 300 mg.

Erfindungsgemäß einsetzbare Arzneimittel, die die Verbindungen der Formel (I) und/oder ihre physiologisch verträglichen Salze enthalten, können enteral, zum Beispiel oral oder rektal, verabreicht werden, zum Beispiel in Form von Pillen, Tabletten, Filmtabletten, Dragees, Granulaten, Hart- und Weichgelatinekapseln, Lösungen wie wäßrigen, alkoholischen oder öligen Lösungen, Säften, Tropfen, Sirupen, Emulsionen oder Suspensionen. Die Verabreichung kann auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Cremes, Pasten, Lotionen, Gelen, Sprays, Puder, Schäumen, Aerosolen oder Lösungen, oder die Verwendung in Form von Implantaten.

Die Herstellung der erfindungsgemäß einsetzbaren pharmazeutischen Präparate kann nach den bekannten Standardverfahren zur Herstellung pharmazeutischer Präparate erfolgen. Dazu werden ein oder mehrere Verbindungen der Formel (I) und/oder ihre physiologisch verträglichen Salze zusammen mit einem oder mehreren festen oder flüssigen galenischen Trägerstoffen und/oder Zusatzstoffen oder Hilfsstoffen und, wenn gewünscht, in Kombination mit anderen Arzneimittelwirkstoffen mit therapeutischer oder prophylaktischer Wirkung, zum Beispiel Herz-Kreislauf-aktiven Arzneimitteln wie etwa Calcium-Antagonisten, ACE-Hemmern, AT1-Rezeptor-Antagonisten, NO-Donoren, Endothelin-Rezeptor-Antagonisten, K-Kanal-Öffnern, Phosphodiesterase-Hemmern, Diuretika oder $\alpha$- und $\beta$-Blockern, in eine geeignete Verabreichungsform bzw. Dosierungsform gebracht, die dann als Arzneimittel in der Humanmedizin oder Veterinärmedizin verwendet werden kann.

Als Trägerstoffe kommen organische oder anorganische Substanzen in Frage, die sich für die enterale (zum Beispiel orale) oder parenterale (zum Beispiel intravenöse) Applikation oder topische Anwendungen eignen und mit

den Wirkstoffen der Formel (I) nicht reagieren, beispielsweise Wasser, pflanzliche Öle, Alkohole wie Ethanol, Isopropanol oder Benzylalkohole, 1,2-Propandiol, Polyethylenglykole, Glycerin-triacetat, Gelatine, Kohlenhydrate wie Lactose oder Stärke, Magnesium-stearat, Talk, Lanolin, Vaseline, Acetonitril, Dimethylformamid, Dimethylacetamid. Zur oralen und rektalen Anwendung dienen insbesondere Arzneiformen wie Tabletten, Dragees, Kapseln, Lösungen, vorzugsweise ölige oder wäßrige Lösungen, Sirupe, Säfte oder Tropfen, ferner Suspensionen oder Emulsionen. Es können auch Gemische von zwei oder mehreren Trägerstoffen eingesetzt werden, zum Beispiel Gemische von zwei oder mehr Lösungsmitteln, insbesondere auch Gemische von einem oder mehreren organischen Lösungsmitteln mit Wasser. Als Zusatzstoffe oder Hilfsstoffe können die pharmazeutischen Präparate zum Beispiel Stabilisierungs- und/oder Netzmittel, Emulgatoren, Salze zum Beispiel zur Beeinflussung des osmotischen Druckes, Gleit-, Konservierungs-, Farb- und Geschmacks- und/ oder Aromastoffe, Puffersubstanzen enthalten. Sie können falls erwünscht auch einen oder mehrere weitere Wirkstoffe enthalten, zum Beispiel ein oder mehrere Vitamine. Die Verbindungen der Formel (I) und/oder deren physiologisch verträgliche Salze können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht.

Die Dosierung des zu verabreichenden Wirkstoffs der Formel (I) und/oder eines physiologisch verträglichen Salz davon bei der erfindungsgemäßen Verwendung hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel (I) weitere Wirkstoffe verabreicht werden. Im allgemeinen ist ein Dosisbereich zur Behandlung der vorgenannten Krankheitsbilder im Menschen von etwa 0.1 mg bis etwa 100 mg pro kg und Tag bei Verabreichung an einen ca. 75 kg schweren Erwachsenen zur Erzielung der angestrebten Wirkung angemessen. Bevorzugt ist ein Dosisbereich von 1 bis 20 mg pro kg und Tag (jeweils mg pro kg Körpergewicht). Die Tagesdosis kann dabei als Einzeldosis verabreicht werden oder in mehrere, zum Beispiel ein, zwei, drei oder vier, Einzeldosen aufgeteilt werden. Sie kann auch kontinuierlich verabreicht werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 300 mg Wirkstoff der Formel (I) und/oder dessen physiologisch verträgliche Salze.

Liste der Abkürzungen:

| | |
|---|---|
| abs. | absolut |
| cGMP | cyclisches 3',5'-Guanosinmonophoshat |
| $CH_2Cl_2$ | Dichlormethan |
| DCI | Desorption Chemical lonisation |
| DMF | N,N-Dimethylformamid |
| EE | Ethylacetat |
| ESI | Electron Spray lonisation |
| FAB | Fast Atom Bombardment |
| Fp | Schmelzpunkt |
| ges. | gesättigt |
| h | Stunde(n) |
| Min. | Minute(n) |
| NO | Stickstoffmonoxid |
| RT | Raumtemperatur |
| THF | Tetrahydrofuran |

Die Erfindung wird durch die nachstehenden Beispiele erläutert, ohne auf diese beschränkt zu sein.

Beispiele:

Beispiel 1

4-Chloro-5-formyl-2-phenyl-1-[[4-[2-(*n*-butyloxycarbonylsulfonamido)-5-isobutyl-3-thienyl]-phenyl]-methyl]-imidazol

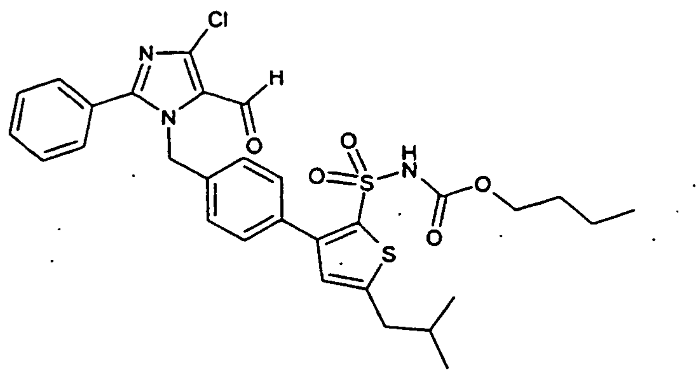

a) 4-Chloro-1-[(4-bromophenyl)-methyl]-5-formyl-2-phemyl-imidazol

Eine Lösung aus 8.0 g (32.0 mmol) 4-Chloro-5-formyl-2-phenyl-imidazol (hergestellt nach Chem. Pharm. Bull. 24, 1976, 960-969) und 5.3 g (32.0 mmol) $K_2CO_3$ in 200 mL abs. DMF wurde für 20 Min. bei RT gerührt. Anschließend wurde eine Lösung von 9.6 g (32.0 mmol) 4-Bromo-benzylbromid in 200 mL abs. DMF hinzugetropft und die Reaktionslösung für 6 h bei RT gerührt. Es wurde im Vakuum eingeengt, der erhaltene Rückstand in EE aufgenommen, mit Wasser, 10%iger $KHSO_4$-, 10 %iger $NaHCO_3$- und ges. Kochsalz-Lösung gewaschen und über $Na_2SO_4$ getrocknet. Chromatographische Reinigung des nach Abzug des EE verbliebenen Rückstandes an $SiO_2$ mit EE/Heptan (1:4) als Laufmittel lieferte 11.5 g der Titelverbindung in Form eines beigen Feststoffes.
Fp: 92 - 95 °C
$R_f$ ($SiO_2$, EE/Heptan 1:4) = 0.24
MS (ESI): m/z = 375/377 $[M+H]^+$

b) 4-Chloro-5-formyl-2-phenyl-1-[[4-[2-(N-*tert*.-butyl-sulfonamido)-5-isobutyl-3-thienyl]-phenyl]-methyl]-imidazol

Bei RT wurde eine Lösung aus 7.2 g (22.6 mmol) 5-Isobutyl-2-[(N-*tert*.-butyl)-sulfonamido]-thiophen-3-boronsäure (bekannt aus EP-A 512 675) in 125 mL Ethanol zu einer Lösung aus 8.5 g (22.6 mmol) der Verbindung aus Beispiel 1a) und 800 mg Tetrakistriphenylphosphin-Palladium-(0) in 100 mL Toluol getropft. Es wurden 26 mL einer 2 M $Cs_2CO_3$-Lösung hinzugefügt und die resultierende Reaktionslösung für 5 h am Rückfluß gerührt. Es wurde zur Trockene eingeengt und der verbliebene Rückstand in EE/Wasser (1:1) aufgenommen. Die organische Phase wurde abgetrennt, mit Wasser gewaschen, über $Na_2SO_4$ getrocknet und eingeengt. Chromatographische Reinigung des Rückstandes an $SiO_2$ mit EE/Heptan (1:4) als Laufmittel ergab 6.7 g der Titelverbindung als weißen Feststoff.
Fp: 104 - 105 °C
$R_f$ ($SiO_2$, EE/Heptan 1:2) = 0.26
MS (ESI): m/z = 570 $[M+H]^+$

c) 4-Chloro-5-formyl-2-phenyl-1-[[4-[2-sulfonamido-5-isobutyl-3-thienyl]-phenyl]-methyl]-imidazol

Eine Lösung aus 3.3 g (5.96 mmol) der Verbindung aus Beispiel 1b) und 3.5 mL (5.96 mmol) Anisol in 33 mL Trifluoressigsäure wurde 48 h bei RT gerührt. Es wurde im Vakuum zur Trockene eingeengt und der Rückstand in EE aufgenommen. Die EE-Lösung wurde mit Wasser gewaschen, über $Na_2SO_4$ getrocknet und eingeengt. Nach chromatographische Reinigung des Rückstandes an $SiO_2$ mit EE/Heptan (1:1) als Laufmittel resultierten 1,52 g der gewünschten Verbindung in Form eines langsam kristallisierenden Feststoffes.
Fp: 118 - 120 °C
$R_f$ ($SiO_2$, EE/Heptan 1:1)= 0.32
MS (ESI): m/z = 515 $[M+H]^+$

d) 4-Chloro-5-formyl-2-phenyl-1-[[4-[2-(*n*-butyloxycarbonylsulfonamido)-5-isobutyl-3-thienyl]-phenyl]-methyl]-imidazol

In einer Argon-Atmosphäre wurde eine Lösung aus 100 mg (0.19 mmol) der Verbindung aus Beispiel 1c) in 1.7 mL abs. Pyridin nacheinander mit 3 mg (0.02 mmol) 4-Pyrrolidinopyridine und 252 µL (0.19 mmol) Chlorameisensäurebutylester versetzt. Die Reaktionslösung wurde 24 h bei RT gerührt. Anschließend wurden 0.7 mL Methanol zugefügt, zur Trockene eingeengt und der Rückstand in EE aufgenommen. Die EE-Lösung wurde dann mit einer 10 %iger ZitronensäureLösung, Wasser und einer ges. Kochsalz-Lösung gewaschen, über $Na_2SO_4$ getrocknet und eingeengt. Die chromatographische Reinigung des nach Abzug des Lösungsmittels erhaltenen Rückstandes an $SiO_2$ mit EE/Heptan (1:1) lieferte schließlich 85 mg der Titelverbindung in Form eines amorphen Feststoffes.
$R_f$ ($SiO_2$, EE/Heptan 1:1) = 0.15
MS (FAB): m/z = 614 $[M+H]^+$

Beispiel 2

5-Formyl-4-methoxy-2-phenyl-1-[[4-[2-(n-butyloxycarbonylsulfonamido)-5-isobutyl-3-thienyl]-phenyl]-methyl]-imidazol

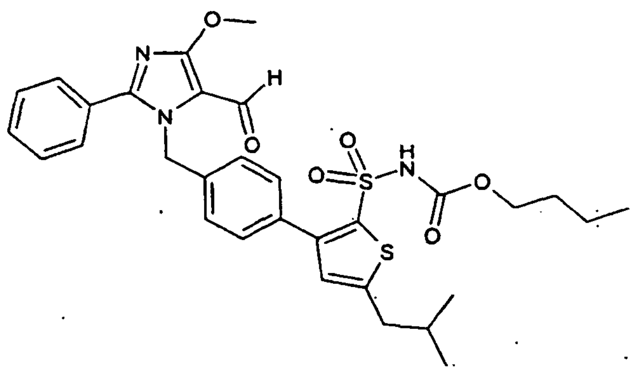

a) 5-Formyl-4-methoxy-2-phenyl-1-[[4-[2-sulfonamido-5-isobutyl-3-thienyl]-phenyl]-methyl]-imidazol

Eine Lösung von 850 mg (1.65 mmol) der Verbindung aus Beispiel 1c) in 25 mL Methanol wurde mit 665 mg (16.53 mmol) NaOH versetzt und 20 h unter Rückfluß gerührt. Die Reaktionslösung wurde eingeengt, der Rückstand in 60 mL EE/Wasser (1:1) aufgenommen, der pH der Lösung durch Zugabe von 1 N Salzsäure auf 6 eingestellt und die organische Phase abgetrennt. Die wässerige Phase wurde 2x mit EE extrahiert und die vereinten organischen Phasen über $Na_2SO_4$ getrocknet. Chromatographische Reinigung des nach Abzug des EE erhaltenen Rückstandes an $SiO_2$ mit EE/Heptan (1:1) als Laufmittel ergab 690 mg der Titelverbindung in Form eines gelben, amorphen Schaumes.
$R_f$ ($SiO_2$, EE/Heptan 1:1) = 0.23
MS (FAB): m/z = 510 $[M+H]^+$

b) 5-Formyl-4-methoxy-2-phenyl-1-[[4-[2-(*n*-butyloxycarbonylsulfonamido)-5-isobutyl-3-thienyl]-phenyl]-methyl]-imidazol

Die Herstellung der Titelverbindung erfolgte durch Umsatz der Verbindung aus Beispiel 2a) mit Chlorameisensäurebutylester nach dem in Beispiel 1d) angeführten Verfahren. Dabei resultierten aus 106 mg (0.21 mmol) der Verbindung aus Beispiel 2a) nach chromatographischer Reinigung an $SiO_2$ mit EE/Heptan (1:1) als Laufmittel 75 mg der gewünschten Verbindung als amorpher Schaum.
$R_f$ ($SiO_2$. EE/Heptan 1:1) = 0.18
MS (ESI): m/z = 610 $[M+H]^+$

Beispiel 3

5-Formyl-4-methoxy-2-phenyl-1-[[4-[2-(*n*-propyloxycarbonylsulfonamido)-5-isobutyl-3-thienyl]-phenyl]-methyl]-imidazol

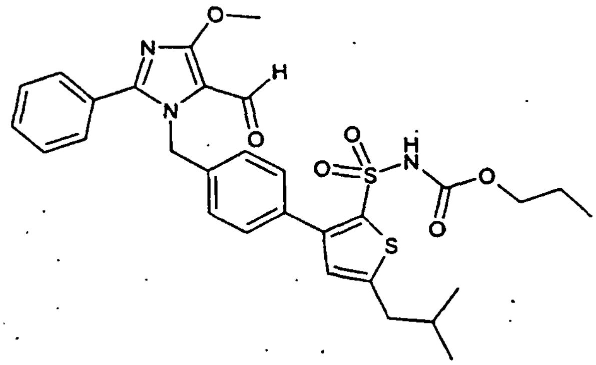

Die Herstellung der Titelverbindung erfolgte durch Umsatz der Verbindung aus Beispiel 2a) mit Chlorameisensäurepropylester nach dem in Beispiel 1d) angeführten Verfahren. Dabei wurden aus 60 mg (0.12 mmol) der Verbindung aus Beispiel 2a) nach chromatographischer Reinigung an $SiO_2$ mit EE/Heptan (1:1) 61 mg der Titelverbindung als amorpher Schaum erhalten.
$R_f$ ($SiO_2$, EE/Heptan 1:1) = 0.13
MS (ESI): m/z = 596 $[M+H]^+$

Beispiel 4

5-Formyl-4-methoxy-2-phenyl-1-[(4-[2-(ethoxycarbonylsulfonamido)-5-isobutyl-3-thienyl]-phenyl]-methyl]-imidazol

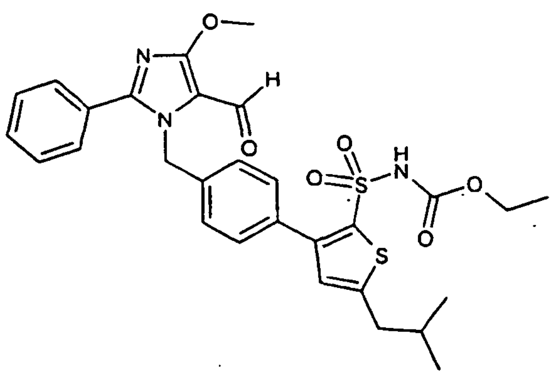

Die Herstellung der Titelverbindung erfolgte durch Umsatz der Verbindung aus Beispiel 2a) mit Chlorameisensäureethylester nach dem in Beispiel 1d) angeführten Verfahren. Dabei wurden aus 60 mg (0.12 mmol) der Verbindung aus Beispiel 2a) nach chromatographischer Reinigung an $SiO_2$ mit EE/Heptan (1:1) 55 mg der Titelverbindung als amorpher Schaum erhalten.
$R_f$ ($SiO_2$, EE/Heptan 1:1) = 0.10
MS (ESI): m/z = 582 $[M+H]^+$

Beispiel 5

5-Formyl-4-methoxy-2-phenyl-1-[[4-[2-(methoxycarbonylsulfonamido)-5-isobutyl-3-thienyl]-phenyl]-methyl]-imidazol

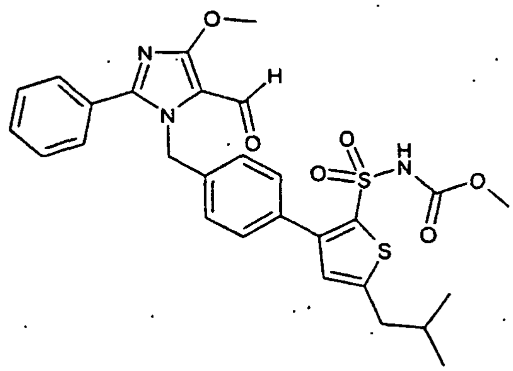

Eine Lösung von 80 mg (0.16 mmol) der Verbindung aus Beispiel 2a), 43.3 mg (0.32 mmol) $K_2CO_3$ und 8.3 mg Dimethylaminopyridin in 6 mL Diethylenglycoldimethylether wurde mit 16.8 µL (0.16 mmol) Dimethyldicarbonat versetzt und anschließend für 1.5 h unter Rückfluß gerührt. Die Reaktionslösung wurde zur Trockene eingeengt und der Rückstand in einer Lösung aus EE und einer 10 %iger $KH_2PO_4$-Lösung (1:1) aufgenommen. Die organische Phase wurde abgetrennt, 2x mit einer 10 %igen $KH_2PO_4$-Lösung gewaschen, über $Na_2SO_4$ getrocknet und eingeengt. Chromatographische Reinigung des Rückstandes an $SiO_2$ mit EE/Heptan (2:1) lieferte 55 mg der Titelverbindung in Form eines amorphen Schaumes.
$R_f$ ($SiO_2$, EE/Heptan 4:1) = 0.23
MS (ESI): m/z = 568 $[M+H]^+$

Beispiel 6

5-Formyl-4-methoxy-2-phenyl-1-[[4-[2-(*n*-butylaminocarbonylsulfonamido)-5-isobutyl-3-thienyl]-phenyl]-methyl]-imidazol

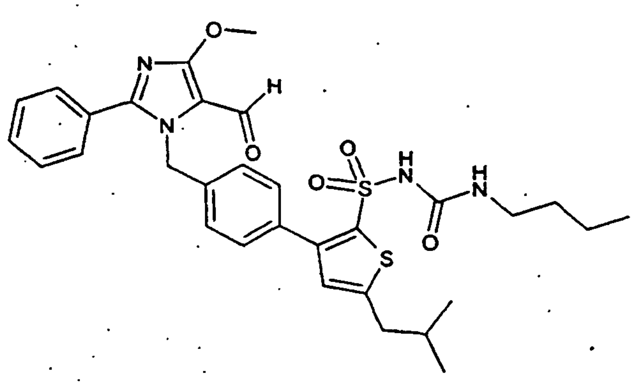

Eine Lösung von 60 mg (0.12 mmol) der Verbindung aus Beispiel 2a) in 2 mL abs. DMF wurde nacheinander mit 48 mg (0.35 mmol) $K_2CO_3$ und 13.2 µL (0.12 mmol) n-Butylisocyanat versetzt und anschließend für 3 h unter Rückfluß gerührt. Der Reaktionslösung wurde nach dem Abkühlen 15 mL einer 10 %igen $KH_2PO_4$-Lösung hinzugefügt und die erhaltene Lösung mehrfach mit EE extrahiert. Die vereinten organischen Phasen wurden über $Na_2SO_4$ getrocknet und eingeengt. Der erhaltene Rückstand wurde mit EE/Diisopropylether versetzt und der ausgefallene Niederschlag abgesaugt. Die Trocknung des Niederschlages im Vakuum ergab 55 mg der Titelverbindung.
Fp: 131 - 133 °C

$R_f$ ($SiO_2$, EE/Heptan 4:1) = 0.30
MS (FAB): m/z = 609 $[M+H]^+$

Beispiel 7

5-Formyl-4-methoxy-2-phenyl-1-[[4-[2-(ethylaminocarbonylsulfonamido)-5-isobutyl-3-thienyl]-phenyl]-methyl]-imidazol

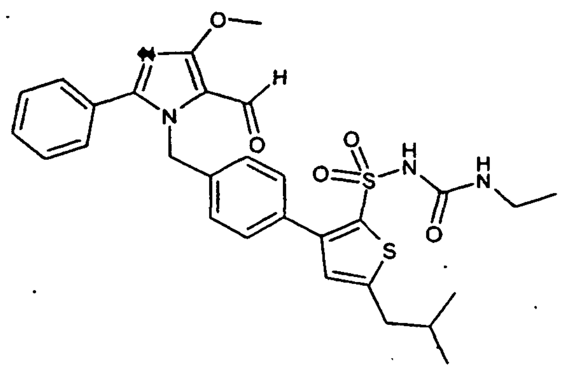

Die Herstellung der Titelverbindung erfolgte durch Umsatz der Verbindung aus Beispiel 2a) mit Ethylisocyanat nach dem in Beispiel 6) angeführten Verfahren. Dabei wurden aus 60 mg (0.12 mmol) der Verbindung aus Beispiel 2a) 46 mg der Titelverbindung gewonnen.
Fp: 105 - 106 °C
$R_f$ ($SiO_2$, EE/Heptan 4:1) = 0.30
MS (ESI): m/z = 581 $[M+H]^+$

Beispiel 8

5-Formyl-4-methoxy-2-phenyl-1-[[4-[2-(methylaminocarbonylsulfonamido)-5-isobutyl-3-thienyl]-phenyl]-methyl]-imidazol

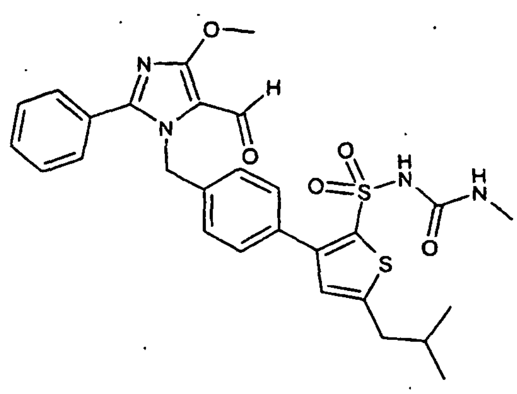

Eine Lösung von 80 mg (0.16 mmol) der Verbindung aus Beispiel 2a) in 1.5 mL DMSO wurde mit 30.4 mg (0.17 mmol) N-Methyl-2,2,2-trichloracetamid und 19.1 mg (0.47 mmol) gepulvertem NaOH versetzt und 1 h bei 80 °C gerührt. Die Reaktionslösung wurde abgekühlt, mit Eis versetzt und der pH durch Zugabe von 2 N Salzsäure auf 4 eingestellt. Der dabei ausgefallene Niederschlag wurde abgesaugt, mit Wasser gewaschen, getrocknet und durch Chromatographie an $SiO_2$ mit EE/Heptan (2:1) als Laufmittel gereinigt. Es wurden 62 mg der Titelverbindung in Form eines weißen Feststoffes gewonnen.

Fp: 102 - 103°C
$R_f$ ($SiO_2$, EE/Heptan 4:1) = 0.14
MS (ESI): m/z = 567 $[M+H]^+$

Beispiel 9

5-Formyl-4-methoxyethoxy-2-phenyl-1-[[4-[2-(n-butyloxycarbonylsulfonamido)-5-isobutyl-3-thienyl]-phenyl]-methyl]-imidazol

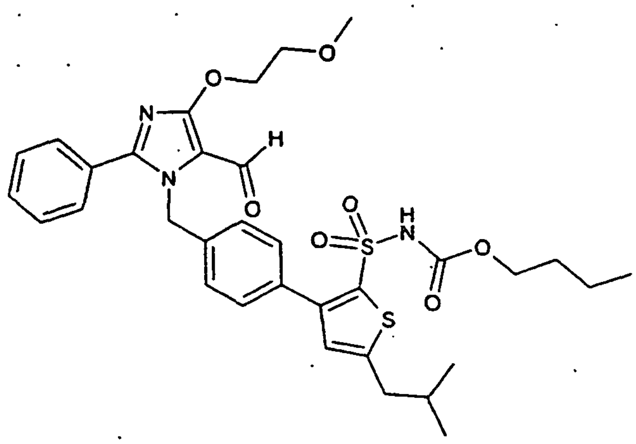

a) 5-Formyl-4-methoxyethoxy-2-phenyl-1-[[4-[2-sulfonamido-5-isobutyl-3-thienyl]-phenyl]-methyl]-imidazol

In einer Argon-Atmosphäre wurde eine Lösung von 200 mg (0.38 mmol) der Verbindung aus Beispiel 1c) in 7.8 mL Ethylenglycolmonomethylether mit 155 mg (3.89 mmol) gepulverten NaOH versetzt und anschließend 5 h bei 80 °C gerührt. Es wurde zur Trockene eingeengt und der erhaltene Rückstand in einer ges. NaHC03-Lösung und EE aufgenommen. Die EE-Phase wurde abgetrennt und die wässrige Lösung mehrfach mit EE extrahiert. Die organischen Phasen wurden vereint, über Na2S04 getrocknet und eingeengt. Die chromatographische Reinigung des verbliebenen Rückstandes an Si02 mit EE/Heptan (1:1) lieferte 140 mg der Titelverbindung als schwach gelbgefärbten Feststoff.
Fp: 91 - 92 °C
$R_f$ ($SiO_2$, EE/Heptan 1:1) = 0.12
MS (FAB): m/z = 554 $[M+H]^+$

b) 5-Formyl-4-methoxyethoxy-2-phenyl-1-[[4-[2-(*n*-butyloxycarbonylsulfonamido)-5-isobutyl-3-thienyl]-phenyl]-methyl]-imidazol

Die Herstellung der Titelverbindung erfolgte durch Umsatz der Verbindung aus Beispiel 9a) mit Chlorameisensäurebutylester nach dem in Beispiel 1d) angeführten Verfahren. Dabei resultierten aus 70 mg (0.13 mmol) der Verbindung aus Beispiel 9a) nach chromatographischer Reinigung an SiO2 mit EE/Heptan (1:1) als Laufmittel 78 mg der Titelverbindung als amorpher Schaum.
$R_f$ ($SiO_2$, EE/Heptan 1:1) = 0.07
MS (ESI): m/z = 654 $[M+H]^+$

Beispiel 10

5-Formyl-4-methoxy-2-phenyl-1-[[4-[2-(*n*-butyloxycarbonylsulfonamido)-5-isobutyl-3-thienyl]-2-chlorphenyl]-methyl]-imidazol

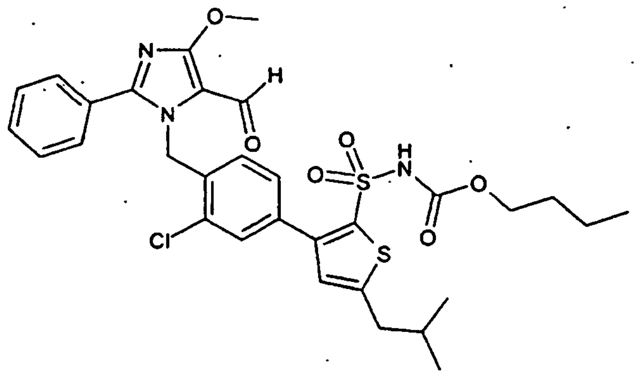

a) 4-Chloro-1-[(4-bromo-2-chlorophenyl)-methyl]-5-formyl-2-phenyl-imidazol

Die Herstellung der Titelverbindung erfolgte durch Umsatz von 4-Chloro-5-formyl-2-phenyl-imidazol mit 4-Bromo-2-chloro-benzylbromid nach dem in Beispiel 1a) angeführten Verfahren. Dabei resultierten aus 2.0 g (9.68 mmol) 4-Chloro-5-formyl-2-phenyl-imidazol 2.6 g der Titelverbindung.
$R_f$ ($SiO_2$, EE/Heptan 1:2) = 0.56
MS (DCI): m/z = 409/411 $[M+H]^+$

b) 4-Chloro-5-formyl-2-phenyl-1-[[4-[2-(N-*tert*.-butyl-sulfonamido)-5-isobutyl-3-thienyl]-2-chlorophenyl]-methyl]-imidazol

Die Titelverbindung wurde durch Umsatz der Verbindung aus Beispiel 10a) und 5-Isobutyl-2-[(N-*tert*.-butyl)-sulfonamido]-thiophen-3-boronsäure nach dem in Beispiel 1b) angeführten Verfahren hergestellt. Dabei wurden aus 2.0 g (4.88 mmol) der Verbindung aus Beispiel 10a) 1.2 g der Titelverbindung in Form eines hellbraunen Öls erhalten.
$R_f$ ($SiO_2$, EE/Heptan 1:2) = 0.47
MS (FAB): m/z = 604 $[M+H]^+$

c) 4-Chloro-5-formyl-2-phenyl-1-[[4-[2-sulfonamido-5-isobutyl-3-thienyl]-2-chlorophenyl]-methyl]-imidazol

Die Herstellung der Titelverbindung erfolgte aus der Verbindung aus Beispiel 10b) nach dem in Beispiel 1c) angeführten Verfahren. Aus 1.2 g (1.99 mmol) der Verbindung aus Beispiel 10b) resultierten 606 mg der Titelverbindung als amorpher, gelber Schaums.
$R_f$ ($SiO_2$, EE/Heptan 1:2) = 0.32
MS (FAB): m/z = 548 $[M+H]^+$

d) 5-Formyl-2-methoxy-2-phenyl-1-[[4-[2-sulfonamido-5-isobutyl-3-thienyl]-2-chlorophenyl]-methyl]-imidazol

Die Titelverbindung wurde aus der Verbindung aus Beispiel 10c) nach dem in Beispiel 2a) angegebenen Verfahren hergestellt. Dabei resultierten aus 400 mg (0.73 mmol) der Verbindung aus Beispiel 10c) 280 mg der Titelverbindung in Form eines gelben, amorphen Schaumes.
Fp: 60°C (Erweichung)
$R_f$ ($SiO_2$, EE/Heptan 1:2) = 0.20
MS (ESI): m/z = 544 $[M+H]^+$

e) 5-Formyl-4-methoxy-2-phenyl-1-[[4-[2-(*n*-butyloxycarbonylsulfonamido)-5-isobutyl-3-thienyl]-2-chlorphenyl]-methyl]-imidazol.

Die Titelverbindung resultierte aus dem Umsatz der Verbindung aus Beispiel 10d) mit Chlorameisensäurebutylester nach dem in Beispiel 1d) angeführten Verfahren. Aus 200 mg (0.37 mmol) der Verbindung aus Beispiel 10d) wurden 167 mg der gewünschten Verbindung in Form eines beigen Feststoffes gewonnen.
Fp: 58°C (Erweichung)
$R_f$ ($SiO_2$, EE/Heptan 1:1) = 0.45
MS (ESI): m/z = 644 $[M+H]^+$

Beispiel 11

5-Formyl-4-methoxy-2-phenyl-1-[[4-[2-(*n*-butyloxycarbonylsulfonamido)-5-isobutyl-3-thienyl]-2-chlorphenyl]-methyl]-imidazol

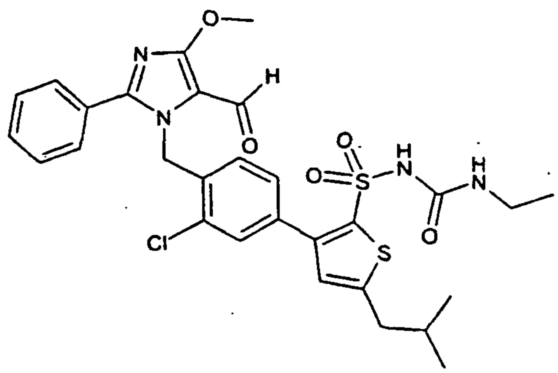

Die Titelverbindung resultierte aus dem Umsatz der Verbindung aus Beispiel 10d) mit Ethylisocyanat nach dem in Beispiel 7) beschriebenen Verfahren. Dabei resultierten aus 74 mg (0.14 mmol) der Verbindung aus Beispiel 10d) nach chromatographischer Reinigung an $SiO_2$ mit $CH_2Cl_2$/Methanol (20:1) 35 mg der Titelverbindung in Form eines weißen Feststoffes.
Fp: 83 °C (Erweichung)
$R_f$ ($SiO_2$, EE/Heptan 1:1) = 0.30
MS (ESI): m/z = 614 $[M+H]^+$

Beispiel 12

4-Chloro-5-formyl-2-phenyl-1-[[4-[2-(*n*-butyloxycarbonylsulfonamido)-5-*n*-propyl-3-thienyl]-phenyl]-methyl]-imidazol

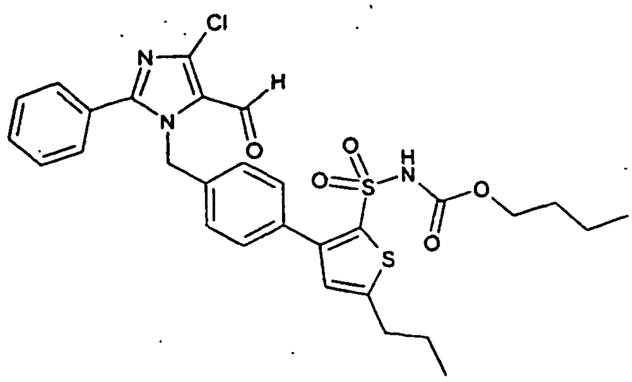

a) 4-Chloro-5-formyl-2-phenyl-1-[[4-[2-(N-*tert*.-butyl-sulfonamido)-5-*n*-propyl-3-thienyl]-phenyl]-methyl]-imidazol

Die Herstellung der Titelverbindung erfolgte durch den Umsatz der Verbindung aus Beispiel 1a) mit 5-*n*-Propyl-2-[(N-*tert*.-butyl)-sulfonamido]-thiophen-3-boronsäure (bekannt aus EP-A 512 675) nach dem in Beispiel 1b) angeführten Verfahren. Dabei wurden aus 4.8 g (13.1 mmol) der Verbindung aus Beispiel 1a) nach chromatographischer Reinigung an $SiO_2$ mit EE/Heptan (1:3) als Laufmittel 2.9 g der Titelverbindung in Form eines weißen Feststoffes erhalten.
Fp: 140 °C
$R_f$ ($SiO_2$, EE/Heptan 1:2) = 0.30
MS (FAB): m/z = 556 $[M+H]^+$

b) 4-Chloro-5-formyl-2-phenyl-1-[[4-[2-sulfonamido-5-*n*-propyl-3-thienyl]-phenyl]-methyl]-imidazol

Die Titelverbindung wurde aus der Verbindung aus Beispiel 12a) nach dem in Beispiel 1c) angeführten Verfahren hergestellt. Aus 1.9 g (3.56 mmol) der Verbindung aus Beispiel 12a) resultierten nach chromatographischer Reinigung an $SiO_2$ mit EE/Heptan (1:2) als Laufmittel 1.1 g der Titelverbindung als weißer Feststoff.
Fp: 93 - 95 °C
$R_f$ ($SiO_2$, EE/Heptan 1:2) = 0.18
MS (ESI): m/z = 500 $[M+H]^+$

c) 4-Chloro-5-formyl-2-phenyl-1-[[4-[2-(*n*-butyloxycarbonylsulfonamido)-5-*n*-propyl-3-thienyl]-phenyl]-methyl]-imidazol

Die Herstellung der Titelverbindung erfolgte durch Umsatz der Verbindung aus Beispiel 12b) mit Chlorameisensäurebutylester nach dem in Beispiel 1d) angeführten Verfahren. Dabei wurden aus 100 mg (0.20 mmol) der Verbindung aus Beispiel 12b) nach chromatographischer Reinigung an $SiO_2$ mit EE/Heptan (1:1), als Laufmittel 90 mg der Titelverbindung erhalten.
$R_f$ ($SiO_2$, EE/Heptan 1:1) = 0.14
MS (ESI): m/z = 600 $[M+H]^+$

Beispiel 13

5-Formyl-4-methoxy-2-phenyl-1-[[4-[2-(*n*-butyloxycarbonylsulfonamido)-5-*n*-propyl-3-thienyl]-phenyl]-methyl]-imidazol

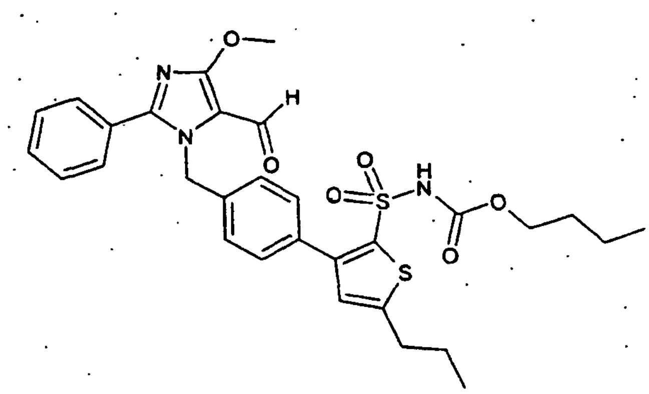

a) 5-Formyl-4-methoxy-2-phenyl-1-[[4-[2-sulfonamido-5-*n*-propyl-3-thienyl]-phenyl]-methyl]-imidazol

Die Herstellung der Titelverbindung erfolgte durch Umsatz der Verbindung aus Beispiel 12b) nach dem in Beispiel 2a) angeführten Verfahren. Es resultierten dabei aus 850 mg (1.70 mmol) der Verbindung aus Beispiel 12b) nach chromatographischer Reinigung an SiO2 mit EE/Heptan (1:2) 460 mg der Titelverbindung in Form eines weißen Feststoffes.
Fp: 85 - 86 °C
$R_f$ ($SiO_2$, EE/Heptan 1:1) = 0.22
MS (ESI): m/z = 496 $[M+H]^+$

b) 5-Formyl-4-methoxy-2-phenyl-1-[[4-[2-(*n*-butyloxycarbonylsulfonamido)-5-*n*-propyl-3-thienyl]-phenyl]-methyl]-imidazol

Die Herstellung der Titelverbindung erfolgte durch Umsatz der Verbindung aus Beispiel 13a) mit Chlorameisensäurebutylester nach dem in Beispiel 1d) angeführten Verfahren. Dabei wurden aus 60 mg (0:12 mmol) der Verbindung aus Beispiel 13a) nach chromatographischer Reinigung an $SiO_2$ mit EE/Heptan (1:1) als Laufmittel 52 mg der Titelverbindung erhalten.
$R_f$ ($SiO_2$, EE/Heptan 1:1) = 0.18
MS (ESI): m/z = 596 $[M+H]^+$

Beispiel 14

5-Formyl-4-methoxy-2-phenyl-1-[[4-[2-(methoxycarbonylsulfonamido)-5-*n*-propyl-3-thienyl]-phenyl]-methyl]-imidazol

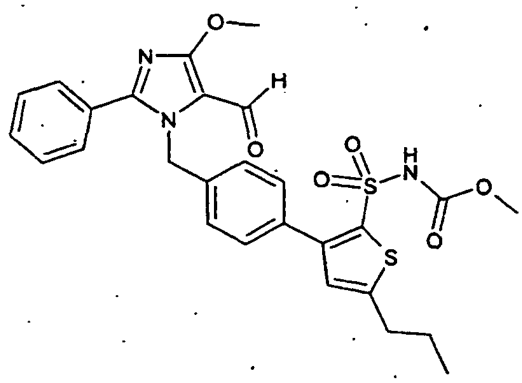

Die Titelverbindung wurde durch Umsatz der Verbindung aus Beispiel 13b) mit Dimethyldicarbonat nach dem in Beispiel 5) angeführten Verfahren hergestellt. Aus 75 mg (0.15 mmol) der Verbindung aus Beispiel 13b) wurden nach Chromatographie an $SiO_2$ mit EE/Heptan (2:1) als Laufmittel 66 mg der Titelverbindung als amorpher Feststoff erhalten.
$R_f$ ($SiO_2$, EE/Heptan 4:1) = 0.18
MS (ESI): m/z = 554 $[M+H]^+$

Beispiel 15

5-Formyl-4-methoxy-2-phenyl-1-[[4-[2-(*n*-butylaminocarbonylsulfonamido)-5-*n*-propyl-3-thienyl]-phenyl]-methyl]-imidazol

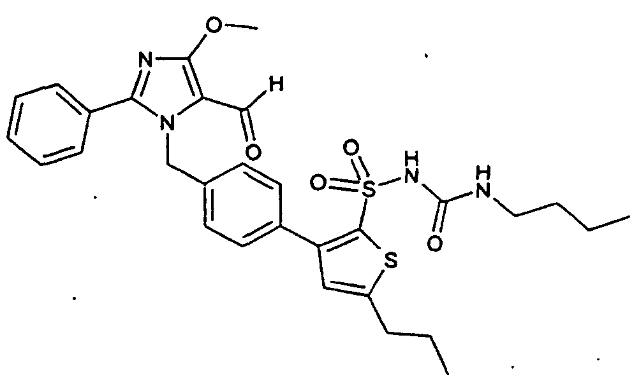

Die Titelverbindung wurde durch Umsatz der Verbindung aus Beispiel 13b) mit *n*-Butylisocyanat nach dem in Beispiel 6) angeführten Verfahren hergestellt. Aus 59 mg (0.12 mmol) der Verbindung aus Beispiel 13b) wurden nach Chromatographie an $SiO_2$ mit EE/Heptan (1:1) als Laufmittel 54 mg der Titelverbindung als amorpher Feststoff erhalten.
$R_f$ ($SiO_2$, EE/Heptan 4:1) = 0.25
MS (ESI): m/z = 595 $[M+H]^+$

Beispiel 16

5-Formyl-4-methoxy-2-phenyl-1-[[4-[2-(methylaminocarbonylsulfonamido)-5-*n*-propyl-3-thienyl]-phenyl]-methyl]-imidazol

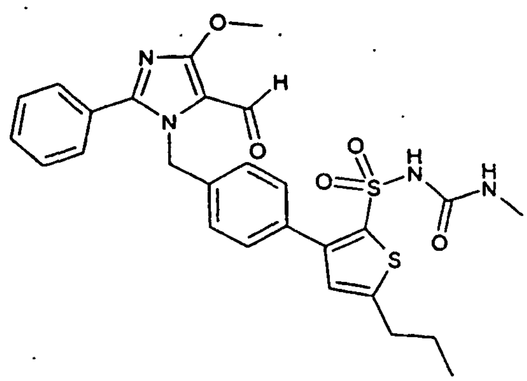

Die Titelverbindung wurde durch Umsatz der Verbindung aus Beispiel 13b) mit N-Methyl-2,2,2-trichloracetamid nach dem in Beispiel 8) angeführten Verfahren hergestellt. Aus 70 mg (0.14 mmol) der Verbindung aus Beispiel 13b) wurden nach Chromatographie an $SiO_2$ mit EE/Heptan (2:1) als Laufmittel 55 mg der Titelverbindung als amorpher Feststoff erhalten.
$R_f$ ($SiO_2$, EE/Heptan 4:1) = 0.15
MS (ESI): m/z = 553 $[M+H]^+$

Beispiel 17:

5-Formyl-4-methoxy-2-phenyl-1-[[4-[2-(ethylaminocarbonylsulfonamido)-5-isobutyl-3-thienyl]-phenyl]-methyl]-imidazol Natriumsalz

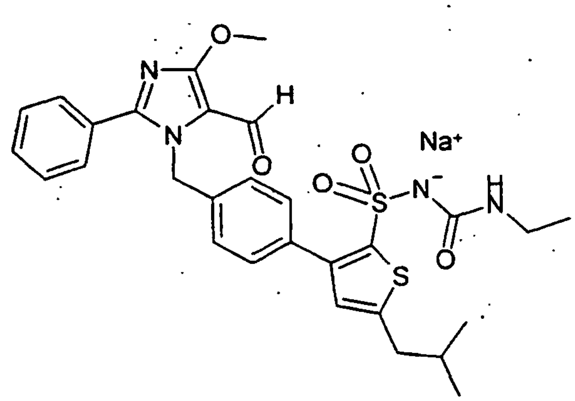

220 mg (0.38 mmol) der Verbindung aus Beispiel 7 wurden mit 3.7 ml einer frisch hergestellten 0.1 molaren Natriummethanolat-Lösung versetzt und die resultierende Lösung 1 h bei Raumtemperatur gerührt. Die Reaktionslösung wurde zur Trockene eingeengt und der erhaltene Rückstand unter leichter Erwärmung in 4 ml Essigsäure-n-butylester gelöst.. Der nach 3-tägiger Aufbewahrung im Kühlschrank auskristallisierende Niederschlag wurde abgesaugt und mit wenig kaltem Essigsäure-n-butylester gewaschen. Trocknung im Hochvakuum lieferte schließlich 120 mg des gewünschten Natriumsalzes.
Fp: 170°C

MS (ESI): m/z = 603 $[M+H]^+$

Beispiel 18

5-Formyl-4-methoxy-2-phenyl-1-[[4-[2-(ethylaminocarbonylsulfonamido)-5-isobutyl-3-thienyl]-phenyl]-methyl]-imidazol L-Lysin-Salz

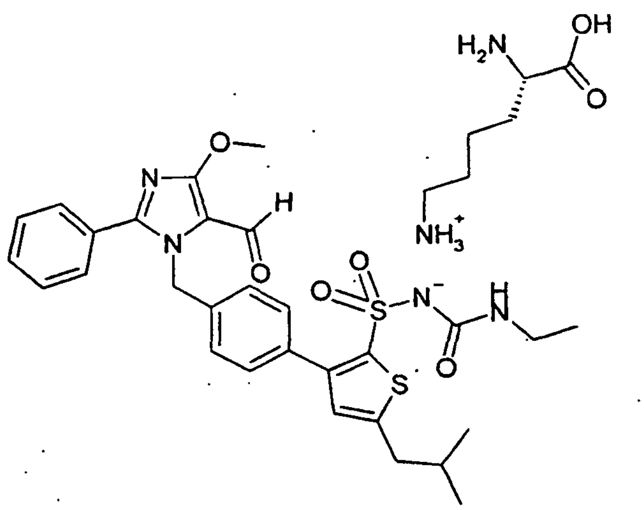

Eine Lösung aus 500 mg (0.86 mmol) der Verbindung aus Beispiel 7 und 125.8 mg (0.86 mmol) L-Lysin in 100 ml Ethanol und 25 ml Wasser wurden 2 h bei RT gerührt. Anschließend wurde zur Trockene eingeengt, der Rückstand in 30 ml Wasser aufgenommen und die erhaltene Lösung gefriergetrocknet. 200 mg vom erhaltenen amorphen Rückstand wurden in 10 ml heißen Toluol gelöst. Nach Aufbewahrung im Kühlschrank für mehrere Tage wurde der auskristallisierende Niederschlag abfiltriert und im Hochvakuum getrocknet. Es resultierten 68 mg der Titelverbindung als blaßgelb gefärbte Kristalle.
Fp: 180 °C
MS (ESI): m/z = 727 $[M+H]^+$

Beispiel 19

5-Formyl-4-methoxy-2-phenyl-1-[[4-[2-(ethylaminocarbonylsulfonamido)-5-isobutyl-3-thienyl]-phenyl]-methyl]-imidazol Tris(hydroxymethyl)aminomethan-Salz

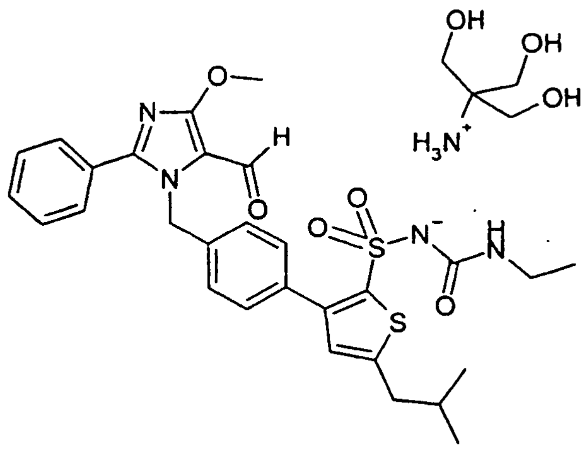

Eine Lösung von 300 mg (0.516 mmol) der Verbindung aus Beispiel 7 und 62.6 mg (0.516 mmol) Tris(hydroxymethyl)aminomethan in 75 ml Ethanol und 15-ml Wasser wurde 2 h bei RT gerührt. Es wurde zur Trockene eingeengt, der Rückstand in Wasser aufgenommen und gefriergetrocknet. Der erhaltenen amorphe Rückstand wurde in 30 mL Essigsäure-n-butylester in der Wärme gelöst. Nach Aufbewahrung der Lösung im Kühlschrank für mehrere Tage wurde der auskristallisierende Niederschlag abgesaugt und im Hochvakuum getrocknet. Es resultierten 120 mg der Titelverbindung in Form blaßgelb gefärbter Kristalle.
Fp.: 144-145°C
MS (ESI): m/z = 702 $[M+H]^+$

In den folgenden Assays (Test 1 und 2) wurde die Affinität der Verbindungen der Formel (I) zu Angiotensin-(1-7)-Bindungsstellen sowie deren agonistischen Eigenschaften an Endothelzellen nachgewiesen:

Test 1: Bindungsassay:

Die Messung der Affinität der Verbindungen der Formel (I) zu Angiotensin-(1-7)-Rezeptoren erfolgte durch Ligandenverdrängungsexperimenten an Membranpräparationen von primären Rinderaorten-Endothelzellen, wie sie beispielsweise auch in Hypertension, 1997; 29[part 2]:388-393 beschrieben sind.

a) Membranpräparation:

Nach Gewinnung von Endothelzellen von Rinderaorten (Test 1, a), wurden die Zellen bis zum Erreichen ihrer Konfluenz in 75 $cm^2$ Kulturflaschen (Becton Dickinson, Heidelberg) kultiviert. Danach wurden die Zellen mit eiskaltem Phosphat-NaCl-EDTA- Puffer (50 mmol/L $NaHPO_4$, 0.15 mol/L NaCl, 5 mmol/L EDTA, pH 7.2) aufgenommen, mit einem Gummischaber abgelöst und zentrifugiert (1500 x g, 5 Min). Das resultierende Zellpellet wurde zur späteren Membranpräparation eingefroren (-80°C).Das aufgetaute Zellpellet wurde in eiskaltem Phosphat-NaCl-EDTA-Puffer homogenisiert (Glass/Teflon Potter, 1000 U/min, 10 strokes). Die Membranisolierung erfolgte durch anschließende Zentrifugation (30 000 x g, 20 Min.) des Zellhomogenates. Das so gewonnene Zellpellet wurde in modifiziertem HEPES-Puffer (10 nmol/L HEPES, 0.1 mol/L NaCl, 5 mmolL $MgCl_2$, pH 7.4) mit Zusatz von 0.2% Rinderserumalbumin und einem Proteasen-Inhibitoren-Cocktail (Complete™, Boehringer Mannheim) resuspendiert. Nach anschließender Proteinbestimmung (nach Lowry) der Membransuspensioh wurde diese sofort für den Ligandenbindungsversuch verwendet.

b) Bindungsexperimente:

Die Versuche wurden auf 96-well Opak-Platten, die mit Durapore-Filtem (0.65 μm Porengröße; Millipore,

Eschborn) bestückt sind, durchgeführt. Vor Beginn des Versuches wurden die Filter mit 1% Rinderserumalbumin für 30 Min. vorbehandelt, um die unspezifische Bindung des radioaktiven Liganden und der kalten Substanzen an das Filtermaterial zu minimieren. Die Inkubation erfolgte in einem Gesamtvolumen von 200 µl: 50 µl $^{125}$J-ANG-(1-7), 20 µl kaltes, nicht-radioaktives ANG-(1-7) oder Testsubstanzen der Formel (I), 30 µl Puffer und 100 µl Membranen (20 µg Protein). Die Bindungsreaktion wurde durch Zugabe des radioaktiven Liganden gestartet. Die Inkubation der Proben erfolgte unter ständigem Schütteln bei Zimmertemperatur für 45 Min. Die Bindungsreaktion wurde mittels Vakuumfiltration (-20 kPa Vakuum; Multiscreen Filtrationssystem, Millipore, Eschborn) beendet. Um die nicht-membrangebundene, freie Radioaktivität vollständig zu entfernen, wurden die Filter 2mal mit 250 µl eiskaltem Phosphat-NaCl-EDTA-Puffer (50 mmol/L $NaHPO_4$, 0.15 mol/L NaCl, 5 mmol/L EDTA, pH 7.2) unter Vakuum gewaschen und dann getrocknet. Der radioaktive Gehalt auf den getrockneten Filtern wurde mittels eines Gammmazählers bestimmt. Für die Kompetitionsversuche (Bestimmung von "Einzelwerten" oder $IC_{50}$-Werten) wurde eine Konzentration von 7.5 bis 10 nmol/L $^{125}$J-ANG-(1-7) (spezifische Aktivität 1500-2100 mCi/mg) eingesetzt, mit und ohne aufsteigenden Konzentrationen der Testsubstanzen der Formel (I). Die unspezifische Bindung wurde jeweils in Gegenwart von 10 µmol/L nicht-radioaktivem ANG-(1-7) gemessen.

c) Ergebnisse:

| Beispiel | $IC_{50}$ [nM] |
|---|---|
| 2a | 20 |
| 2b | 30 |
| 4 | 5 |
| 7 | 20 |

Die Ergebnisse belegen die hohe Affinität der Verbindungen der Formel (I) zum Angiotensin-(1-7)-Rezeptoren an Endothelzellen.
Bezüglich ANGII-Rezeptoren des $AT_1$- und des $AT_2$-Typs weisen die Verbindungen der Formel (I) dabei keine bzw. nur eine vernachlässigbare (> $10^{-6}$ M) Affinität auf.

Test 2: Funktionaler Assay:

Die Messung der stimulierenden Wirkung der Verbindungen der Formel (I) auf die Produktion von intrazellulärem cGMP als Marker für die Produktion und Freisetzung von NO in Endothelzellen erfolgte an primär kultivierten Endothelzellen von Rinderaorten, wie sie beispielweise in J. Pharmacol. Exp. Ther. 1992, 262, 729-733 beschrieben ist.

a) Zellkulturen:

Nach enzymatischer Digestion (Dispase II; Boehringer, Mannheim) der Endothelzellen von der Rinderaorta wurden die Endothelzellen in Kulturmedium (Dulbecco's modifiziertes Eagle's Ham's F 12 Medium 1:1 mit Penicillin (10 U/L), Streptomycin (10 µg/L), L-Glutamin (1mmol/L), Glutathion and L-(+)-Ascorbinsäure (jeweils 5 mg/L) und hitzeinaktiviertes fetales Kälberserum (20%)) aufgenommen, 1 mal gewaschen (Zentrifugation bei 170 x g, 10 min) und in Kulturmedium resuspendiert. Die so gewonnene Zellsuspension wurde in 6-well Platten (Nunc Intermed, Wiesbaden) ausgesät (~250 µg Protein oder 3 x $10^{-5}$ Zellen per well), mit Kulturmedium aufgefüllt und bei 37°C in einem befeuchteten, und mit 95%$O_2$-5%$CO_2$ begasten Inkubator gehalten.

b) cGMP-Bestimmungen:

Nach Erreichen der Konfluenz (6-8 Tage nach der Aussaat) wurde das Kulturmedium entfernt und der Zellmonolayer 2mal mit warmer HEPES/Tyrode's Lösung gewaschen. Danach wurden die Zellen für 15 Min. bei 37°C in HEPES/Tyrode's Lösung, die IBMX (3-Isobutyl-1-methylxanthin, $10^{-4}$ mol/L, Serva, Heidelberg) enthält, vorinkubiert. Die Inkubation wurde durch Zugabe von SOD (Superoxiddismutase von Rindererythrozyten, 3 x $10^{-7}$ mol/L, Serva, Heidelberg) und den Testsubstanzen der Formel (I) in den angegeben Konzentrationen gestartet. Nach der entsprechenden Inkubationszeit wurde das Inkubationsmedium abgsaugt, die zurückbleibenden Zellen sofort in 1 N Ameisensäure-Aceton (v/v, 15:85) extrahiert und abgeschabt. Die erhaltene Suspension wurde ultrabeschallt (10 Sek.) und dann abzentrifugiert (3000 x g, 10 Min). Für die Bestimmung von cGMP mittels Radioimmunoassay (New England

Nuclear, Bosten, MA) wurde der Überstand lyophylisiert und in Natrium-Acetat-Puffer (0.05 mol/L;pH 6.2) aufgenommen. Der Gehalt (pmol) an intrazellulärem cGMP wurde auf mg Zellprotein bezogen.

c) Ergebnisse:

| Beispiel | $EC_{50}$ [µM] |
|---|---|
| 2a | 0.5 |
| 2b | 0.3 |
| 4 | 0.1 |
| 7 | 0.5 |

Die Ergebnisse belegen die ägonistische Wirkung der Verbindungen der Formel (I) auf Angiotensin-(1-7) -Rezeptoren.
Die Wirkung der erfindungsgemäßen Verbindung auf die Produktion von cGMP als Marker für die NO-Synthese und -Freisetzung wird dabei durch Vorinkubation mit einem Angiotensin 11 Rezeptor-Antagonisten sowohl des $AT_1$-Subtypes wie EXP3174 oder des $AT_2$-Subtypes wie PD 123,319 nicht beeinflußt. Im Gegensatz dazu wird der beschriebene stimulierende Effekt der erfindungsgemäßen Verbindung auf das cGMP durch Vorinkubation mit einen selektiven Antagonisten der Angiotensin-(1-7)-Rezeptoren, [D-$Ala^7$]-Angiotensin-(1-7), der beispielweise in Brain Res. Bull. 1994, 35, 293-298 beschrieben ist, gehemmt, welches die Spezifität dieses funktionalen Effektes belegt.
Die Wirkung der Verbindungen der Formel (I) auf das Herz wurde im Modell des isolierten, arbeitenden Rattenherzen (Test 3) nachgewiesen, welches beispielweise in J. Cardiovasc. Pharmacol. 1986, 8 [Suppl. 10]: S91-S99 beschrieben ist.

Test 3: Isolierte, arbeitende Rattenherzen

a) Methode:

Isolierte Herzen von Wistar-Kyoto-Ratten (280 - 300 g Körpergewicht) werden nach der Methode von Langendorff mit einer Sauerstoff-gesättigten (95 % $O_2$, 5 % $CO_2$), nicht-rezirkulierenden, modifizierten Krebs-Henseleit-Pufferlösung (118 mmol/L NaCl, 4.7 mmol/L KCl; 2.5 mmol/L $CaCl_2$, 1.6 mmol/L $MgSO_4$, 24.9 mmol/L $NaHCO_3$, 1.2 mmol/L $KH_2PO_4$, 5.5 mmol/L Glucose und 2.0 mmol/L Natriumpyruvat) mit einem konstanten Perfusionsdruck von 60 mmHg perfundiert. Zur Messung des koronaren Flusses diente ein in die Pulmonalarterie plazierter Katheder mit einem elektromagnetischen Meßkopf. Nach einer 15-minütigen Aquilibrierungsperiode wird das Herz in den Arbeitmodus überführt, in dem eine Vorlast (pre-load) von 15 mmHg und eine Nachlast (after-load) von 60 mmHg eingestellt wird. Die Arbeitslast der Herzen bleibt während des gesamten Versuchszeit über 90 Minuten konstant. Fluß- und Druck-Signale für die Auswertung werden mittels eines PLUGSYS-Meßsystems (Hugo Sachs Elektronik) registriert. Die Auswertung der Daten erfolgt mit einer Sammelfrequenz von 500 Hz, alle 2 Sekunden gemittelt, mit der Software Aquire Plus VI.21f (PO-NE-MAH).

b) Ergebnisse:

Bei Perfusion der Herzen (n = 4) mit einer Konzentration von $10^{-6}$ mol/L der Verbindung aus Beispiel 2 wurden im Vergleich zu Kontrollherzen (n = 4) folgende Werte für den koronaren Fluß ermittelt:

1. Behandelte Herzen:

| Koronarfluß [mL/Min.] | Zeit [Min.] |
|---|---|
| 8.92 ± 0.68 | 0 |
| 11.29 ± 0.90 | 5 |
| 12.17 ± 0.74 | 10 |
| 12.22 ± 0.10 | 15 |

2. Kontroll-Herzen:

| Koronarfluß [mL/Min.] | Zeit [Min.] |
|---|---|
| 8.98 ± 0.59 | 0 |
| 8.94 ± 0.52 | 5 |
| 9.04 ± 0.70 | 10 |
| 8.91 ± 0.44 | 15 |

Die Herzfrequenz blieb während des gesamten Versuch in beiden Gruppen unverändert.

Diese signifikante Erhöhung des koronaren Flusses an isolierten, arbeitenden Rattenherzen belegt die kardioprotektive Wirkung der Verbindungen der Formel (I).

Die Wirkung der Verbindungen der Formel (I) auf die Kollagen-induzierte Thrombozytenaggregation wurde an humanen plättchenreichen Plasma untersucht, die z.B. in G.V. Born et al., Nature 1962 beschrieben ist.

Test 4:

a) Methode:

Humanes plättchenreiches Plasma (RPR) von 6 Blutspendern wurde 20 min. mit der Testverbindung bei 37 °C inkubiert, dann mit Collagen aktiviert und die maximale Aggregation der Thrombozyten in % via Lichttransmission quantifiziert.

b) Ergebnis:

Bei Inkubation des plättchenreichen Plasmas mit 30 μM der Verbindung aus Beispiel 2 wurden folgende Werte für die Thrombozytenaggregation (n=6) ermittelt:

Collagen (= maximale Aggregation): 92 ± 2.7 % Aggregation
Collagen + 30 μM der Verbindung aus Beispiel 2: 52 ± 5.7 % Aggregation

Diese signifikante Hemmung der Thrombozytenaggregation von humanen plättchenreichen Plasma belegt die anti-thrombotische Wirkung der Verbindungen der Formel (I).

**Patentansprüche**

**1.** Verbindungen der Formel (I),

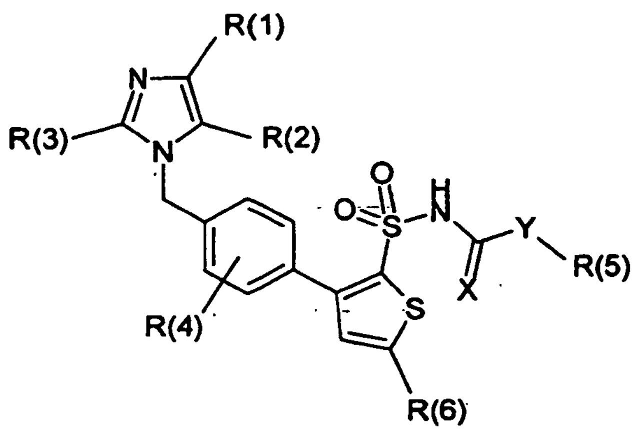

(I)

in denen die angeführten Reste die folgende Bedeutung haben:

R(1)
1. Halogen;
2. Hydroxy;
3. $(C_1-C_4)$-Alkoxy;
4. $(C_1-C_8)$-Alkoxy, wobei 1 bis 6 Kohlenstoffatome gegen die Heteroatome O, S oder NH ausgetauscht sind;
5. $(C_1-C_4)$-Alkoxy, substituiert mit einem gesättigtem cyclischen Ether;
6. O-$(C_1-C_4)$-Alkenyl;
7. O-$(C_1-C_4)$-Alkyl-Aryl; und
8. Phenoxy, unsubstituiert oder substituiert mit einem Substituenten aus der Reihe Halogen, $(C_1-C_3)$-Alkyl, $(C_1-C_3)$-Alkoxy oder Trifluormethyl;

R(2)
1. CHO;
2. COOH; und
3. CO-O-$(C_1-C_4)$-Alkyl;

R(3)
1. $(C_1-C_4)$-Alkyl; und
2. Aryl;

R(4)
1. Wasserstoff;
2. Halogen; und
3. $(C_1-C_4)$-Alkyl;

X
1. Sauerstoff;
2. Schwefel;

Y
1. Sauerstoff; und
2. -NH-;

R(5)
1. Wasserstoff;
2. $(C_1-C_6)$-Alkyl; und
3. $(C_1-C_4)$-Alkyl-Aryl;

wobei R(5) nur dann auch Wasserstoff sein kann, wenn Y die unter 2. angeführte Bedeutung besitzt;

R(6)
1. $(C_1-C_5)$-Alkyl;

in allen ihren stereoisomeren Formen und Mischungen davon in allen Verhältnissen, und ihre physiologisch verträglichen Salze;
ausgenommen Verbindungen der Formel (1), in denen gleichzeitig R(1) für Halogen steht und R(2) die unter 2. und 3. angeführte Bedeutung hat.

**2.** Verbindungen der Formel (1) gemäß Anspruch 1, in der bedeuten:

R(1)
1. Chlor;
2. Hydroxy;
3. Methoxy, Ethoxy, Propyloxy;
4. Methoxyethoxy, Methoxypropoxy;
5. Allyloxy; und
6. Phenoxy;

R(4)
1. Wasserstoff; und
2. Chlor;

R(5)
1. Wasserstoff; und
2. $(C_1-C_4)$-Alkyl;

R(6) n-Propyl und 2-Isobutyl;

und die übrigen Reste wie in Anspruch 1 definiert sind, in allen ihren stereoisomeren Formen und Mischungen davon, und ihre physiologisch verträglichen Salze.

**3.** Verbindungen der Formel (I) gemäß Anspruch 1 oder 2, in der bedeuten:

R(1) Halogen; ($C_1$-$C_4$)-Alkoxy; oder ($C_1$-$C_8$)-Alkoxy, wobei 1 bis 6 Kohlenstoffatome gegen die Heteroatome O, S oder NH ausgetauscht sind;

R(2) CHO;

R(3) Aryl;

R(4) Halogen oder Wasserstoff;

R(5) ($C_1$-$C_6$)-Alkyl;

R(6) ($C_1$-$C_5$)-Alkyl;

X Sauerstoff; und

Y Sauerstoff oder -NH-;

in allen ihren stereoisomeren Formen und Mischungen davon, und ihre physiologisch verträglichen Salze.

**4.** Verbindungen der Formel (I) gemäß einem oder mehreren der Ansprüche 1 bis 3, worin diese eine Verbindungen der Formel (II) darstellen,

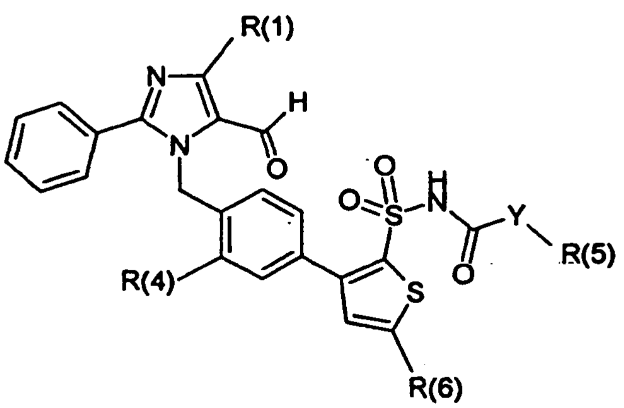

(II)

in der die Reste R(1), R(4), R(5), R(6) und Y die in einem oder mehreren der Ansprüche 1 bis 3 angeführte Bedeutung haben, in allen ihren stereoisomeren Formen und Mischungen davon, und ihre physiologisch verträglichen Salze.

**5.** Verbindungen der Formel (I) gemäß einem oder mehreren der Ansprüche 1 bis 4, in denen R(1) ($C_1$-$C_4$)-Alkoxy oder ($C_1$-$C_8$)-Alkoxy, wobei 1 bis 6 Kohlenstoffatome gegen die Heteroatome O, S oder NH, ausgetauscht sind bedeutet und die übrigen Reste wie in Anspruch 1 bis 4 definiert sind, in allen ihren stereoisomeren Formen und Mischungen davon, und ihre physiologisch verträglichen Salze.

**6.** Verbindungen der Formel (I) gemäß einem oder mehreren der Ansprüche 1 bis 5, in denen R(2) CHO bedeutet und die übrigen Reste wie in Anspruch 1 bis 5 definiert sind,
in allen ihren stereoisomereri Formen und Mischungen davon, und ihre physiologisch verträglichen Salze.

**7.** Verbindungen der Formel (I) gemäß einem oder mehreren der Ansprüche 1 bis 6, in denen X O bedeutet und die

übrigen Reste wie in Anspruch 1 bis 6 definiert sind, in allen ihren stereoisomeren Formen und Mischungen davon, und ihre physiologisch verträglichen Salze.

8. Verbindungen der Formel (I) gemäß einem oder mehreren der Ansprüche 1 bis 7, **dadurch gekennzeichnet, dass** diese
4-Chloro-5-formyl-2-phenyl-1-[[4-[2-(n-butyloxycarbonylsulfonamido)-5-isobutyl-3-thienyl]-phenyl]-methyl]-imidazol;
5-Formyl-4-methoxy-2-phenyl-1-[[4-[2-(n-butyloxycarbonylsulfonamido)-5-isobutyl-3-thienyl]-phenyl]-methyl]-imidazol;
5-Formyl-4-methoxy-2-phenyl-1-[[4-[2-(n-propyloxycarbonylsulfonamido)-5-isobutyl-3-thienyl]-phenyl]-methyl]-imidazol
5-Formyl-4-methoxy-2-phenyl-1-[[4-[2-(ethoxycarbonylsulfonamido)-5-isobutyl-3-thienyl]-phenyl]-methyl]-imidazol;
5-Formyl-4-methoxy-2-phenyl-1-[[4-[2-(methoxycarbonylsulfonamido)-5-isobutyl-3-thienyl]-phenyl]-methyl]-imidazol;
5-Formyl-4-methoxy-2-phenyl-1-[[4-[2-(n-butylaminocarbonylsulfonamido)-5-isobutyl-3-thienyl]-phenyl]-methyl]-imidazol;
5-Formyl-4-methoxy-2-phenyl-1-[[4-[2-(ethylaminocarbonylsulfonamido)-5-isobutyl-3-thienyl]-phenyl]-methyl]-imidazol;
5-Formyl-4-methoxy-2-phenyl-1-[[4-[2-(ethylaminocarbonylsulfonamido)-5-isobutyl-3-thienyl]-phenyl]-methyl]-imidazol Natriumsalz;
5-Formyl-4-methoxy-2-phenyl-1-[[4-[2-(ethylaminocarbonylsulfonamido)-5-isobutyl-3-thienyl]-phenyl]-methyl]-imidazol L-Lysin-Salz;
5-Formyl-4-methoxy-2-phenyl-1-[[4-[2-(ethylaminocarbonylsulfonamido)-5-isobutyl-3-thienyl]-phenyl]-methyl]-imidazol Tris(hydroxymethyl)aminomethan-Salz;
5-Formyl-4-methoxy-2-phenyl-1-[[4-[2-(methylaminocarbonylsulfonamido)-5-isobutyl-3-thienyl]-phenyl]-methyl]-imidazol;
5-Formyl-4-methoxyethoxy-2-phenyl-1-[[4-[2-(n-butyloxycarbonylsulfonamido)-5-isobutyl-3-thienyl]-phenyl]-methyl]-imidazol;
5-Formyl-4-methoxy-2-phenyl-1-[[4-[2-(n-butyloxycarbonylsulfonamido)-5-isobutyl-3-thienyl]-2-chlorphenyl]-methyl]-imidazol;
5-Formyl-4-methoxy-2-phenyl-1-[[4-[2-(n-butyloxycarbonylsulfonamido)-5-isobutyl-3-thienyl]-2-chlorphenyl]-methyl]-imidazol;
4-Chloro-5-formyl-2-phenyl-1-[[4-[2-(n-butyloxycarbonylsulfonamido)-5-n-propyl-3-thienyl]-phenyl]-methyl]-imidazol;
5-Formyl-4-methoxy-2-phenyl-1-[[4-[2-(n-butyloxycarbonylsulfonamido)-5-n-propyl-3-thienyl]-phenyl]-methyl]-imidazol;
5-Formyl-4-methoxy-2-phenyl-1-[[4-[2-(methoxycarbonylsulfonamido)-5-n-propyl-3-thienyl]-phenyl]-methyl]-imidazol;
5-Formyl-4-methoxy-2-phenyl-1-[[4-[2-(n-butylaminocarbonylsulfonamido)-5-n-propyl-3-thienyl]-phenyl]-methyl]-imidazol; oder
5-Formyl-4-methoxy-2-phenyl-1-[[4-[2-(methylaminocarbonylsulfonamido)-5-n-propyl-3-thienyl]-phenyl]-methyl]-imidazol;
sind, sowie deren physiologisch verträglichen Salze.

9. Verbindungen der Formel (X)

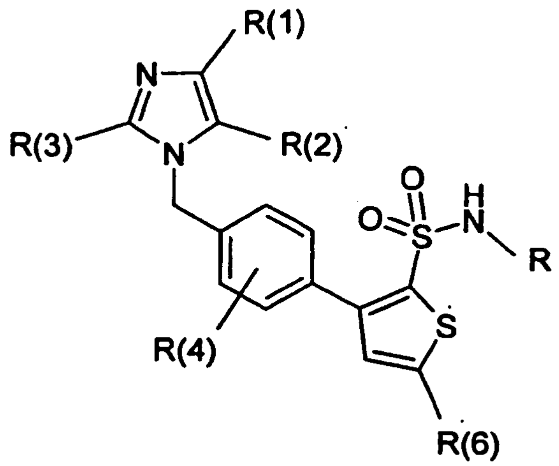

(X)

in denen R Wasserstoff oder ($C_1$-$C_6$)-Alkyl bedeutet und die Reste R(1), R(2), R(3), R(4), R(6) wie in einem oder mehreren der Ansprüche 1 bis 8 definiert sind, in allen ihren stereoisomeren Formen und Mischungen davon, und ihre physiologisch verträglichen Salze.

**10.** Verbindungen der Formel (I) oder (X) gemäß einem oder mehreren der Ansprüche 1 bis 9 zur Anwendung als Arzneimittel.

**11.** Pharmazeutische Zubereitung, **gekennzeichnet durch** einen wirksamen Gehalt einer Verbindung der Formel (I) oder (X) nach einem oder mehreren der Ansprüche 1 bis 9 und/oder eines physiologisch verträglichen Salzes davon.

**12.** Verwendung von Verbindungen der Formel (I) oder (X) gemäß einem oder mehreren des Anspruche 1-9 zur Herstellung eines Medikaments zur Behandlung und/oder Prophylaxe von Bluthochdruck; Herzhypertrophie, Herzinsuffizienz, koronaren Herzerkrankungen wie der Angina Pectoris, Herzinfarkt, vaskulärer Restenose nach Angioplastie, Kardiomyopathien, endothelialer Dysfunktion bzw. endothelialer Schädigungen, z.B. als Folge atherosklerotischer Prozesse oder Diabetis mellitus, sowie von arteriellen und venösen Thrombosen.

**13.** Verbindungen der Formel (I) oder (X) gemäß einem oder mehreren der Ansprüche 1 bis 9 zur Verwendung in der Therapie und/oder Prophylaxe von Krankheiten, die primär oder sekundär durch eine reduzierte Produktion und/oder Freisetzung der vasorelaxierenden, antithrombotischen und kardioprotektiven Botenstoffe cyclisches 3',5'-Guanosinmonophoshat (cGMP) und Stickstoffmonoxid (NO) verursacht oder zumindest mitverursacht werden.

**14.** Verbindungen der Formel (I) oder (X) gemäß einem oder mehreren der Ansprüche 1 bis 9 zur Verwendung in der Therapie und/oder Prophylaxe von Bluthochdruck, Herzhypertrophie, Herzinsuffizienz, koronaren Herzerkrankungen wie der Angina Pectoris, Herzirifarkt, vaskulärer Restenose nach Angioplastie, Kardiomyopathien, endothelialer Dysfunktion bzw. endothelialer Schädigungen, z.B. als Folge atherosklerotischer Prozesse oder Diabetis mellitus, sowie von arteriellen und venösen Thrombosen.

**Claims**

**1.** Compounds of the formula (I)

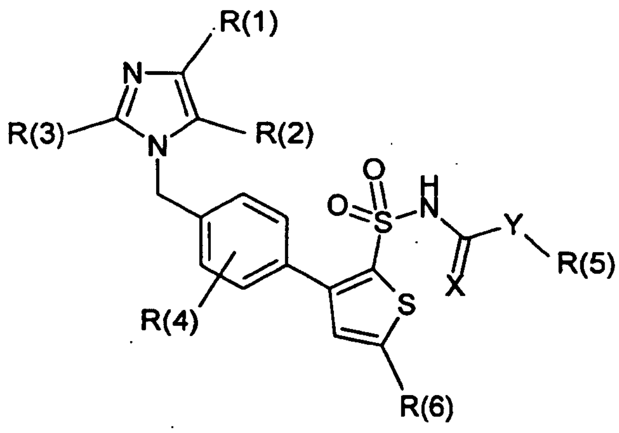

(I)

in each of which the radicals mentioned have the following meaning:

R(1)
1. halogen;
2. hydroxyl;
3. $(C_1-C_4)$-alkoxy;
4. $(C_1-C_8)$-alkoxy, 1 to 6 carbort atoms being replaced by the heteroatoms O, S or NH,
5. $(C_1-C_4)$-alkoxy, substituted by a saturated cyclic ether;
6. O-$(C_1-C_4)$-alkenyl;
7. O-$(C_1-C_4)$-alkylaryl; and
8. phenoxy, unsubstituted or substituted by a substituent from the group consisting of halogen, $(C_1-C_3)$-alkyl, $(C_1-C_3)$-alkoxy or trifluoromethyl;

R(2)
1. CHO;
2. COOH; and
3. CO-O-$(C_1-C_4)$-alkyl;

R(3)
1. $(C_1-C_4)$-alkyl; and
2. aryl;

R(4)
1. hydrogen;
2. halogen; and
3. $(C_1-C_4)$-alkyl;

X
1. oxygen;
2. sulfur;

Y
1. oxygen; and
2. -NH-;

R(5)
1. hydrogen;
2. $(C_1-C_6)$-alkyl; and
3. $(C_1-C_4)$-alkylaryl;

where R(5) can only also be hydrogen if Y has the meaning mentioned under 2.;

R(6)
1. $(C_1-C_5)$-alkyl;

in all their stereoisomeric forms and mixtures thereof in all ratios, and their physiologically tolerable salts; excluding compounds of the formula (I) in which, simultaneously, R(1) is halogen and R(2) has the meaning mentioned under 2. and 3.

**2.** Compounds of the formula (I) as claimed in claim 1, in which:

R(1) is
1. chlorine;
2. hydroxyl;
3. methoxy, ethoxy, propyloxy;
4. methoxyethoxy, methoxypropoxy;
5. allyloxy; and
6. phenoxy;

R(4) is
1. hydrogen; and
2. chlorine;

R(5) is
1. hydrogen; and
2. ($C_1$-$C_4$)-alkyl;

R(6) is n-propyl and 2-isobutyl;

and the other radicals are as defined in claim 1, in all their stereoisomeric forms and mixtures thereof, and their physiologically tolerable salts.

**3.** Compound of the formula (I) as claimed in claim 1 or 2, in which:

R(1) is halogen; ($C_1$-$C_4$)-alkoxy; or ($C_1$-$C_8$)-alkoxy, where 1 to 6 carbon atoms are replaced by the heteroatoms O, S or NH;

R(2) is CHO;

R(3) is aryl;

R(4) is halogen or hydrogen;

R(5) is ($C_1$-$C_6$)-alkyl;

R(6) is ($C_1$-$C_5$)-alkyl;

X is oxygen; and

Y is oxygen or -NH-;

in all their stereoisomeric forms and mixtures thereof, and their physiologically tolerable salts.

**4.** Compound of the formula (I) as claimed in one or more of claims 1 to 3, wherein these constitute compounds of the formula (II)

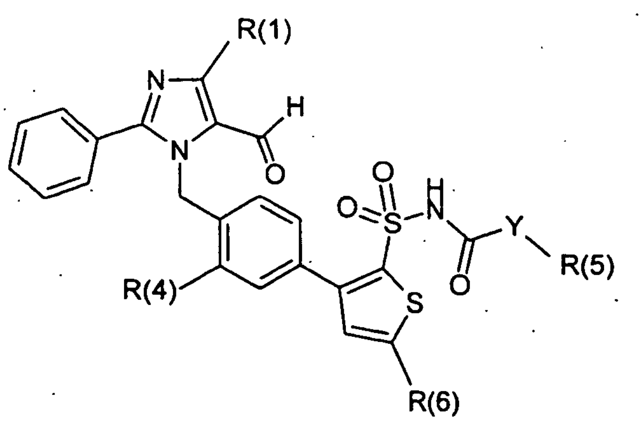

(II)

in which the radicals R(1), R(4), R(5), R(6) and Y have the meaning mentioned in one or more of claims 1 to 3, in all their stereoisomeric forms and mixtures thereof, and their physiologically tolerable salts.

**5.** Compound of the formula (I) as claimed in one or more of claims 1 to 4, in each of which R(1) is ($C_1$-$C_4$)-alkoxy or ($C_1$-$C_8$)-alkoxy, where 1 to 6 carbon atoms are replaced by the heteroatoms O, S or NH, and the other radicals are as defined in claim 1 to 4,
in all their stereoisomeric forms and mixtures thereof, and their physiologically tolerable salts.

**6.** Compound of the formula (I) as claimed in one or more of claims 1 to 5, in each of which R(2) is CHO and the other radicals are as defined in claim 1 to 5,
in all their stereoisomeric forms and mixtures thereof, and their physiologically tolerable salts.

**7.** Compound of the formula (I) as claimed in one or more of claims 1 to 6, in each of which X is O and the other radicals are as defined in claim 1 to 6, in all their stereoisomeric forms and mixtures thereof, and their physiologically tolerable salts.

**8.** Compound of the formula (I) as claimed in one or more of claims 1 to 7, **characterized in that** these
4-chloro-5-formyl-2-phenyl-1-[[4-[2-(n-butyloxycarbonylsulfonamido)-5-isobutyl-3-thienyl]phenyl]methyl]imidazole;
5-formyl-4-methoxy-2-phenyl-1-[[4-[2-(n-butyloxycarbonylsulfonamido)-5-isobutyl-3-thienyl]phenyl]methyl]imidazole;
5-formyl-4-methoxy-2-phenyl-1-[[4-[2-(n-propyloxycarbonylsulfonamido)-5-isobutyl-3-thienyl]phenyl]methyl]imidazole
5-formyl-4-methoxy-2-phenyl-1-[[4-[2-(ethoxycarbonylsulfonamido)-5-isobutyl-3-thienyl]phenyl]methyl]imidazole;
5-formyl-4-methoxy-2-phenyl-1-[[4-[2-(methoxycarbonylsulfonamido)-5-isobutyl-3-thienyl]phenyl]methyl]imidazole;
5-formyl-4-methoxy-2-phenyl-1-[[4-[2-(n-butylaminocarbonylsulfonamido)-5-isobutyl-3-thienyl]phenyl]methyl]imidazole;
5-formyl-4-methoxy-2-phenyl-1-[[4-[2-(ethylaminocarbonylsulfonamido)-5-isobutyl-3-thienyl]phenyl]methyl]imidazole;
5-formyl-4-methoxy-2-phenyl-1-[[4-[2-(ethylaminocarbonylsulfonamido)-5-isobutyl-3-thienyl]phenyl]methyl]imidazole sodium salt;
5-formyl-4-methoxy-2-phenyl-1-[[4-[2-(ethylaminocarbonylsulfonamido)-5-isobutyl-3-thienyl]phenyl]methyl]imidazole L-lysine salt;
5-formyl-4-methoxy-2-phenyl-1-[[4-[2-(ethylaminocarbonylsulfonamido)-5-isobutyl-3-thienyl]phenyl]methyl]imidazole tris(hydroxymethyl)aminomethane salt;
5-formyl-4-methoxy-2-phenyl-1-[[4-[2-(methylaminocarbonylsulfonamido)-5-isobutyl-3-thienyl]phenyl]methyl]imidazole;
5-formyl-4-methoxyethoxy-2-phenyl-1-[[4-[2-(n-butyloxycarbonylsulfonamido)-5-isobutyl-3-thienyl]phenyl]methyl] imidazole;
5-formyl-4-methoxy-2-phenyl-1-[[4-[2-(n-butyloxycarbonylsulfonamido)-5-isobutyl-3-thienyl]-2-chlorophenyl]methyl]imidazole;
5-formyl-4-methoxy-2-phenyl-1-[[4-[2-(n-butyloxycarbonylsulfonamido)-5-isobutyl-3-thienyl]-2-chlorophenyl]methyl]imidazole;
4-chloro-5-formyl-2-phenyl-1-[[4-[2-(n-butyloxycarbonylsulfonamido)-5-n-propyl-3-thienyl]phenyl]methyl]imidazole;
5-formyl-4-methoxy-2-phenyl-1-[[4-[2-(n-butyloxycarbonylsulfonamido)-5-n-propyl-3-thienyl]phenyl]methyl]imidazole;
5-formyl-4-methoxy-2-phenyl-1-[[4-[2-(methoxycarbonylsulfonamido)-5-n-propyl-3-thienyl]phenyl]methyl]imidazole;
5-formyl-4-methoxy-2-phenyl-1-[[4-[2-(n-butylaminocarbonylsulfonamido)-5-n-propyl-3-thienyl]phenyl]methyl]imidazole; or
5-formyl-4-methoxy-2-phenyl-1-[[4-[2-(methylaminocarbonylsulfonainido)-5-n-propyl-3-thienyl]phenyl]methyl]imidazole;
and also physiologically tolerable salts thereof.

**9.** Compound of the formula (X)

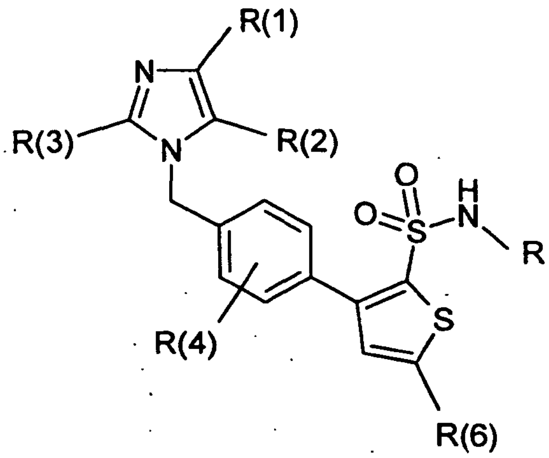

(X)

in each of which R is hydrogen or ($C_1$-$C_6$)-alkyl and the radicals R(1), R(2), R(3), R(4), R(6) are as defined in one or more of claims 1 to 8, in all their stereoisomeric forms and mixtures thereof, and their physiologically tolerable salts.

**10.** Compound of the formula (I) or, (X) as claimed in one or more of claims 1 to 9 for use as a pharmaceutical.

**11.** A pharmaceutical preparation **characterized** an efficacious content of a compound of the formula (I) or (X) as claimed in one or more of claims 1 to 9 and/or of a physiologically tolerable salt thereof.

**12.** The use of compounds of the formula (I) or (X) as claimed in one or more of claims 1-9 for the manufacture of a medicament for the treatment and/or prophylaxis of high blood pressure, cardiac hypertrophy, cardiac insufficiency, coronary heart diseases such as angina pectoris, cardiac infarct, vascular restenosis after angioplasty, cardiomyopathies, endothelial dysfunction or endothelial damage, e.g. as a result of arteriosclerotic processes or diabetes mellitus, and also of arterial and venous thromboses.

**13.** Compound of the formula (I) or (X) as claimed in one or more of claims 1 to 9 for use in the therapy and/or prophylaxis of illnesses which are primarily or secondarily caused or at least partly caused by reduced production and/or release of the vasorelaxant, antithrombotic and cardio-protective messengers cyclic 3',5'-guanosine monophosphate (cGMP) and nitrogen monoxide (NO).

**14.** Compound of the formula (I) or (X) as claimed in one or more of claims 1 to 9 for use in the therapy and/or prophylaxis of high blood pressure, cardiac hypertrophy, cardiac insufficiency, coronary heart diseases such as angina, pectoris, cardiac infarct, vascular restenosis after angioplasty, cardiomyopathies, endothelial dysfunction or endothelial damage, e.g. as a result of arteriosclerotic processes or diabetes mellitus, and also of arterial and venous thromboses.

## Revendications

**1.** Composés de formule (I)

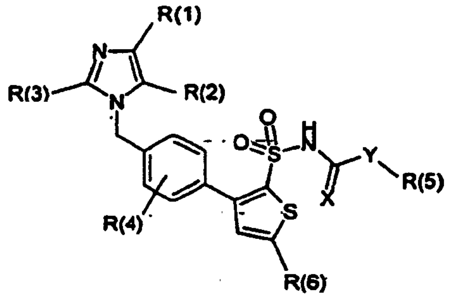

(I).

dans laquelle les radicaux : significations suivantes :

R(1) représente

1. un atome d'halogène ;
2. un groupe hydroxy ;
3. un groupe alcoxy en $C_1$-$C_4$ ;
4. un groupe alcoxy en $C_1$-$C_8$ dans lequel 1 à 6 atomes de carbone sont remplacés par les hétéroatomes O, S ou NH ;
5. un groupe alcoxy en $C_1$-$C_4$ substitué par un éther cyclique saturé ;
6. un groupe O-alcényle en $C_1$-$C_4$ ;
7. un groupe O-alkyl($C_1$-$C_4$)-aryle ; ou
8. un groupe phénoxy non substitué ou portant un substituant choisi dans l'ensemble constitué par un atome d'halogène, un groupe alkyle en $C_1$-$C_3$, un groupe alcoxy en $C_1$-$C_3$ et le groupe trifluorométhyle ;

R(2) représente

1. CHO ;
2. COOH ; ou
3. un groupe CO-O-alkyle($C_1$-$C_4$) ;

R(3) représente

1. un groupe alkyle en $C_1$-$C_4$ ; ou
2. un groupe aryle ;

R(4) représente

1. un atome d'hydrogène ;
2. un atome d'halogène ; ou
3. un groupe alkyle en $C_1$-$C_4$ ;

X représente

1. un atome d'oxygène ;
2. un atome de soufre ;

Y représente

1. un atome d'oxygène ; ou
2. -NH- ;

R(5) représente

1. un atome d'hydrogène ;

2. un groupe alkyle en $C_1$-$C_6$ ; ou
3. un groupe alkyl($C_1$-$C_4$)-aryle ;

R(5) ne pouvant être un atome d'hydrogène que lorsque Y a la signification indiquée en 2. ;

R(6) représente

1. un groupe alkyle en $C_1$-$C_5$ ;

sous toutes leurs formes stéréoisomères et en tous mélanges de celles-ci en tous rapports, et leurs sels physiologiquement acceptables ;
à l'exception des composés de formule (I) dans lesquels simultanément R(1) représente un atome d'halogène et R(2) a la signification indiquée en 2. et 3.

**2.** Composés de formule (I) selon la revendication 1, dans lesquels :

R(1) représente

1. un atome de chlore ;
2. le groupe hydroxy ;
3. le groupe méthoxy, éthoxy, propyloxy ;
4. le groupe méthoxyéthoxy, méthoxypropoxy ;
5. le groupe allyloxy ; ou
6. le groupe phénoxy ;

R(4) représente

1. un atome d'hydrogène ; ou
2. un atome de chlore ;

R(5) représente

1. un atome d'hydrogène ; ou
2. un groupe alkyle en $C_1$-$C_4$ ;

R(6) représente
le groupe n-propyle ou 2-isobutyle ;

et les autres radicaux sont définis comme dans la revendication 1, sous toutes leurs formes stéréo-isomères et en tous mélanges de celles-ci, et leurs sels physiologiquement acceptables.

**3.** Composés de formule (I) selon la revendication 1 ou 2, dans lesquels :

R(1) représente

1. un atome d'halogène ; un groupe alcoxy en $C_1$-$C_4$ ou alcoxy en $C_1$-$C_8$ dans lequel 1 à 6 atomes de carbone sont remplacés par les hétéroatomes 0, S ou NH ;

R(2) représente CHO ;

R(3) représente un groupe aryle ;

R(4) représente un atome d'hydrogène ou d'halogène ;

R(5) représente un groupe alkyle en $C_1$-$C_6$ ;

R(6) représente un groupe alkyle en $C_1$-$C_5$ ;

X représente un atome d'oxygène ; et

Y représente un atome d'oxygène ou -NH- ;

sous toutes leurs formes stéréoisomères et en tous mélanges de celles-ci, et leurs sels physiologiquement acceptables.

**4.** Composés de formule (I) selon une ou plusieurs des revendications 1 à 3, ceux-ci représentant des composés de formule (II),

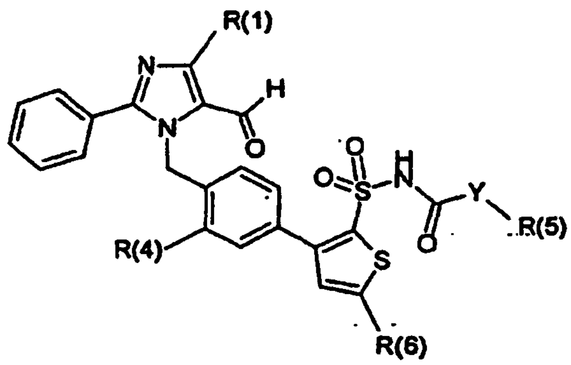

(II)

dans laquelle les radicaux R(1), R(4), R(5), R(6) et Y ont les significations indiquées dans une ou plusieurs des revendications 1 à 3,
sous toutes leurs formes stéréoisomères et en tous mélanges de celles-ci, et leurs sels physiologiquement acceptables.

**5.** Composés de formule (I) selon une ou plusieurs des revendications 1 à 4, dans lesquels R(1) représente un groupe alcoxy en $C_1$-$C_4$ ou alcoxy en $C_1$-$C_8$ dans lequel 1 à 6 atomes de carbone sont remplacés par les hétéroatomes O, S ou NH, et les autres radicaux sont définis comme dans les revendications 1 à 4,
sous toutes leurs formes stéréoisomères et en tous mélanges de celles-ci, et leurs sels physiologiquement acceptables.

**6.** Composés de formule (I) selon une ou plusieurs des revendications 1 à 5, dans lesquels R(2) représente CHO et les autres radicaux sont définis comme dans les revendications 1 à 5,
sous toutes leurs formes stéréoisomères et en tous mélanges de celles-ci, et leurs sels physiologiquement acceptables.

**7.** Composés de formule (I) selon une ou plusieurs des revendications 1 à 6, dans lesquels X représente O et les autres radicaux sont définis comme dans les revendications 1 à 6, sous toutes leurs formes stéréoisomères et en tous mélanges de celles-ci, et leurs sels physiologiquement acceptables.

**8.** Composés de formule (I) selon une ou plusieurs des revendications 1 à 7, **caractérisés en ce que** ces composés sont les suivants :

4-chloro-5-formyl-2-phényl-1-[[4-[2-(n-butyloxycarbonylsulfonamido)-5-isobutyl-3-thiényl]phényl]-méthyl]imidazole,

5-formyl-4-méthoxy-2-phényl-1-[[4-[2-(n-butyloxycarbonylsulfonamido)-5-isobutyl-3-thiényl]phényl]-méthyl] imidazole,

5-formyl-4-méthoxy-2-phényl-1-[[4-[2-(n-propyloxycarbonylsulfonamido)-5-isobutyl-3-thiényl]phényl]-méthyl] imidazole,

5-formyl-4-méthoxy-2-phényl-1-[[4-[2-(éthoxycarbonylsulfonamido)-5-isobutyl-3-thiényl]phényl]-méthyl]imidazole,

5-formyl-4-méthoxy-2-phényl-1-[[4-[2-(méthoxycarbonylsulfonamido)-5-isobutyl-3-thiényl]phényl]-méthyl] imidazole,

5-formyl-4-méthoxy-2-phényl-1-[[4-[2-(n-butylaminocarbonylsulfonamido)-5-isobutyl-3-thiényl]phényl]méthyl]imidazole,

5-formyl-4-méthoxy-2-phényl-1-[[4-[2-(éthylaminocarbonylsulfonamido)-5-isobutyl-3-thiényl]phényl]-méthyl] imidazole,

5-formyl-4-méthoxy-2-phényl-1-[[4-[2-(éthylaminocarbonylsulfonamido)-5-isobutyl-3-thiényl]phényl]-méthyl] imidazole, sel de sodium

5-formyl-4-méthoxy-2-phényl-1-[[4-[2-(éthylaminocarbonylsulfonamido)-5-isobutyl-3-thiényl]phényl]-méthyl] imidazole, sel de L-lysine

5-formyl-4-méthoxy-2-phényl-1-[[4-[2-(éthylaminocarbonylsulfonamido)-5-isobutyl-3-thiényl]phényl]-méthyl] imidazole, sel de tris(hydroxyméthyl)aminométhane

5-formyl-4-méthoxy-2-phényl-1-[[4-[2-(méthylaminocarbonylsulfonamido)-5-isobutyl-3-thiényl]phényl]-méthyl]imidazole,

5-formyl-4-méthoxyéthoxy-2-phényl-1-[[4-[2-(n-butyloxycarbonylsulfonamido)-5-isobutyl-3-thiényl]phényl] méthyl]imidazole,

5-formyl-4-méthoxy-2-phényl-l-[[4-[2-(n-butyloxycarbonylsulfonamido)-5-isobutyl-3-thiényl]-2-chlorophényl] méthyl]imidazole,

5-formyl-4-méthoxy-2-phényl-1-[[4-[2-(n-butyloxycarbonylsulfonamido)-5-isobutyl-3-thiényl]-2-chlorophényl] méthyl]imidazole,

4-chloro-5-formyl-2-phényl-1-[[4-[2-(n-butyloxycarbonylsulfonamido)-5-n-propyl-3-thiényl]phényl]-méthyl]imidazole,

5-formyl-4-méthoxy-2-phényl-1-[[4-[2-(n-butyloxycarbonylsulfonamido)-5-n-propyl-3-thiényl]phényl]-méthyl] imidazole,

5-formyl-4-méthoxy-2-phényl-1-[[4-[-2-(méthoxycarbonylsulfonamido)-5-n-propyl-3-thiényl]phényl]-méthyl] imidazole,

5-formyl-4-méthoxy-2-phényl-1-[[4-[-2-(n-butylaminocarbonylsulfonamido)-5-n-propyl-3-thiényl]-phényl]méthyl]imidazole et

5-formyl-4-méthoxy-2-phényl-1-[[4-[-2-(méthylaminocarbonylsulfonamido)-5-n-propyl-3-thiényl]-phényl]méthyl]imidazole,

ainsi que leurs sels physiologiquement acceptables.

**9.** Composés de formule (X)

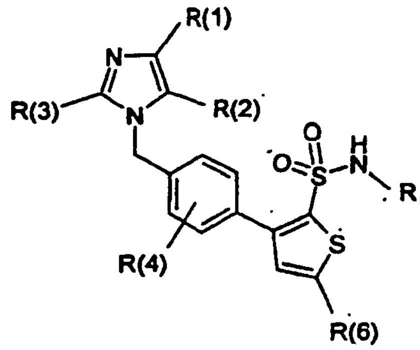

(X)

dans laquelle R représente un atome d'hydrogène ou un groupe alkyle en $C_1$-$C_6$ et les radicaux R(1), R(2), R(3), R(4), R(6) sont définis comme dans une ou plusieurs des revendications 1 à 8, sous toutes leurs formes stéréoisomères et en tous mélanges de celles-ci, et leurs sels physiologiquement acceptables.

**10.** Composés de formule (I) ou (X) selon une ou plusieurs des revendications 1 à 9, pour utilisation en tant que médicament.

**11.** Préparation pharmaceutique, **caractérisée par** une teneur efficace en un composé de formule (I) ou (X) selon une ou plusieurs des revendications 1 à 9 et/ou en un sel physiologiquement acceptable d'un tel composé.

**12.** Utilisation de composés de formule (I) ou (X) selon une ou plusieurs des revendications 1 à 9, pour la fabrication d'un médicament destiné au traitement et/ou à la prophylaxie de l'hypertension artérielle, de l'hypertrophie cardiaque, de l'insuffisance cardiaque, de cardiopathies coronariennes telles que l'angine de poitrine, l'infarctus du myocarde, de la resténose vasculaire après angioplastie, de cardiomyopathies, d'un dysfonctionnement endothélial ou d'endommagements endothéliaux, par exemple comme conséquence de processus athéroscléreux ou du diabète sucré, ainsi que de thromboses artérielles et veineuses.

**13.** Composés de formule (I) ou (X) selon une ou plusieurs des revendications 1 à 9, pour utilisation dans le traitement et/ou la prophylaxie de maladies dont la cause ou au moins la cause simultanée, primaire ou secondaire, est une production réduite et/ou une libération réduite des substances messagères vasorelaxantes, antithrombotiques et cardioprotectrices 3', 5'-guanosine monophosphate cyclique (cGMP) et monoxyde d'azote (NO).

**14.** Composés de formule (I) ou (X) selon une ou plusieurs des revendications 1 à 9, pour utilisation dans le traitement et/ou la prophylaxie de l'hypertension artérielle, de l'hypertrophie cardiaque, de l'insuffisance cardiaque, de cardiopathies coronariennes telles que l'angine de poitrine, l'infarctus du myocarde, de la resténose vasculaire après angioplastie, de cardiomyopathies, d'un dysfonctionnement endothélial ou d'endommagements endothéliaux, par exemple comme conséquence de processus athéroscléreux ou du diabète sucré, ainsi que de thromboses artérielles et veineuses.